# EUROPEAN PATENT APPLICATION

(11) **EP 3 985 124 A1**
(43) Date of publication of application: **20.04.2022**
(21) Application number: 21191144.1
(22) Date of filing: 18.09.2014
(51) Int. Cl.: C12Q 1/68, C12N 15/00, C07H 21/04

(54) **MULTIPLEX GUIDE RNAS**

(30) Priority: 26.12.2013 US 201361921007 P; 23.01.2014 US 201461930782 P; 14.03.2014 US 201414211117; 14.03.2014 WO PCT/US2014/029068; 14.03.2014 WO PCT/US2014/028630; 14.03.2014 US 201414029304; 23.04.2014 WO PCT/US2014/035162
(62) Divisional of application: 14875819.6
(71) Applicant: The General Hospital Corporation, Boston, MA 02114 (US)
(72) Inventor: TSAI, Shengdar, Charlestown, MA 02129 (US); JOUNG, Keith J., Winchester, MA 01890 (US)
(74) Representative: Forresters IP LLP

(57) **Abstract**

Methods and constructs for the multiplex expression of highly active CRISPR guide RNAs (gRNAs) from RNA Polymerase II and III promoters, optionally in mammalian cells.

SEQUENCE LISTING

<110> THE GENERAL HOSPITAL CORPORATION

<120> MULTIPLEX GUIDE RNAS

<130> 40174-0012WO1

<140> PCT/US14/56416
<141> 2014-09-18

<150> PCT/US2014/035162
<151> 2014-03-23

<150> 14/211,117
<151> 2014-03-14

<150> PCT/US2014/029068
<151> 2014-03-14

<150> PCT/US2014/028630
<151> 2014-03-14

<150> PCT/US2014/029304
<151> 2014-03-14

<150> 61/921,007
<151> 2013-12-26

<160> 54

<170> PatentIn version 3.5

<210> 1
<211> 20
<212> DNA
<213> Artificial Sequence

<220>
<223> Description of Artificial Sequence: Synthetic
      oligonucleotide

<400> 1
gttcactgcc gtataggcag                                                   20


<210> 2
<211> 28
<212> DNA
<213> Artificial Sequence

<220>
<223> Description of Artificial Sequence: Synthetic
      oligonucleotide

<400> 2
gttcactgcc gtataggcag ctaagaaa                                          28


<210> 3
<211> 4
<212> PRT
<213> Artificial Sequence

<220>
<223> Description of Artificial Sequence: Synthetic
      peptide

<400> 3
Gly Gly Gly Ser
1


<210> 4
<211> 342
<212> RNA
<213> Artificial Sequence

<220>
<223> Description of Artificial Sequence: Synthetic
      polynucleotide


<220>
<221> modified_base
<222> (1)..(20)
<223> a, c, u or g and this region may encompass 17-20 nucleotides
      wherein some positions may be absent

<220>
<221> modified_base
<222> (43)..(342)
<223> a, c, u or g and this region may encompass 0-300 nucleotides
      wherein some positions may be absent

<400> 4
nnnnnnnnnn nnnnnnnnnn guuuuagagc uaugcuguuu ugnnnnnnnn nnnnnnnnnn      60

nnnnnnnnnn nnnnnnnnnn nnnnnnnnnn nnnnnnnnnn nnnnnnnnnn nnnnnnnnnn     120

nnnnnnnnnn nnnnnnnnnn nnnnnnnnnn nnnnnnnnnn nnnnnnnnnn nnnnnnnnnn     180

nnnnnnnnnn nnnnnnnnnn nnnnnnnnnn nnnnnnnnnn nnnnnnnnnn nnnnnnnnnn     240

nnnnnnnnnn nnnnnnnnnn nnnnnnnnnn nnnnnnnnnn nnnnnnnnnn nnnnnnnnnn     300

nnnnnnnnnn nnnnnnnnnn nnnnnnnnnn nnnnnnnnnn nn                        342

<210> 5
<211> 32
<212> RNA
<213> Artificial Sequence

<220>
<223> Description of Artificial Sequence: Synthetic
      oligonucleotide


<220>
<221> modified_base
<222> (1)..(20)
<223> a, c, u or g and this region may encompass 17-20 nucleotides
      wherein some positions may be absent

<400> 5
nnnnnnnnnn nnnnnnnnnn guuuuagagc ua                                    32

<210> 6
<211> 42
<212> RNA
<213> Artificial Sequence

<220>
<223> Description of Artificial Sequence: Synthetic
      oligonucleotide

<220>
<221> modified_base
<222> (1)..(20)
<223> a, c, u or g and this region may encompass 17-20 nucleotides
      wherein some positions may be absent

<400> 6
nnnnnnnnnn nnnnnnnnnn guuuuagagc uaugcuguuu ug                    42

<210> 7
<211> 36
<212> RNA
<213> Artificial Sequence

<220>
<223> Description of Artificial Sequence: Synthetic
      oligonucleotide

<220>
<221> modified_base
<222> (1)..(20)
<223> a, c, u or g and this region may encompass 17-20 nucleotides
      wherein some positions may be absent

<400> 7
nnnnnnnnnn nnnnnnnnnn guuuuagagc uaugcu                            36

<210> 8
<211> 362
<212> RNA
<213> Artificial Sequence

<220>
<223> Description of Artificial Sequence: Synthetic
      polynucleotide

<220>
<221> modified_base
<222> (1)..(20)
<223> a, c, u or g and this region may encompass 17-20 nucleotides
      wherein some positions may be absent

<220>
<221> modified_base
<222> (63)..(362)
<223> a, c, u or g and this region may encompass 0-300 nucleotides
      wherein some positions may be absent

<400> 8
nnnnnnnnnn nnnnnnnnnn guuuuagagc uagaaauagc aaguuaaaau aaggcuaguc      60

cgnnnnnnnn nnnnnnnnnn nnnnnnnnnn nnnnnnnnnn nnnnnnnnnn nnnnnnnnnn     120

nnnnnnnnnn nnnnnnnnnn nnnnnnnnnn nnnnnnnnnn nnnnnnnnnn nnnnnnnnnn     180

nnnnnnnnnn nnnnnnnnnn nnnnnnnnnn nnnnnnnnnn nnnnnnnnnn nnnnnnnnnn     240

nnnnnnnnnn nnnnnnnnnn nnnnnnnnnn nnnnnnnnnn nnnnnnnnnn nnnnnnnnnn     300

nnnnnnnnnn nnnnnnnnnn nnnnnnnnnn nnnnnnnnnn nnnnnnnnnn nnnnnnnnnn     360

nn                                                                    362


<210> 9
<211> 375
<212> RNA
<213> Artificial Sequence

<220>
<223> Description of Artificial Sequence: Synthetic
      polynucleotide


<220>
<221> modified_base
<222> (1)..(20)
<223> a, c, u or g and this region may encompass 17-20 nucleotides
      wherein some positions may be absent

<220>
<221> modified_base
<222> (76)..(375)
<223> a, c, u or g and this region may encompass 0-300 nucleotides
      wherein some positions may be absent

<400> 9
nnnnnnnnnn nnnnnnnnnn guuuuagagc uaugcugaaa agcauagcaa guuaaaauaa      60

ggcuaguccg uuaucnnnnn nnnnnnnnnn nnnnnnnnnn nnnnnnnnnn nnnnnnnnnn     120

nnnnnnnnnn nnnnnnnnnn nnnnnnnnnn nnnnnnnnnn nnnnnnnnnn nnnnnnnnnn     180

nnnnnnnnnn nnnnnnnnnn nnnnnnnnnn nnnnnnnnnn nnnnnnnnnn nnnnnnnnnn     240

nnnnnnnnnn nnnnnnnnnn nnnnnnnnnn nnnnnnnnnn nnnnnnnnnn nnnnnnnnnn     300

nnnnnnnnnn nnnnnnnnnn nnnnnnnnnn nnnnnnnnnn nnnnnnnnnn nnnnnnnnnn     360

nnnnnnnnnn nnnnn                                                      375


<210> 10
<211> 387
<212> RNA
<213> Artificial Sequence

<220>
<223> Description of Artificial Sequence: Synthetic
      polynucleotide

<220>
<221> modified_base
<222> (1)..(20)
<223> a, c, u or g and this region may encompass 17-20 nucleotides
      wherein some positions may be absent

<220>
<221> modified_base
<222> (88)..(387)
<223> a, c, u or g and this region may encompass 0-300 nucleotides
      wherein some positions may be absent

<400> 10
nnnnnnnnnn nnnnnnnnnn guuuuagagc uaugcuguuu uggaaacaaa acagcauagc      60

aaguuaaaau aaggcuaguc cguuaucnnn nnnnnnnnnn nnnnnnnnnn nnnnnnnnnn     120

nnnnnnnnnn nnnnnnnnnn nnnnnnnnnn nnnnnnnnnn nnnnnnnnnn nnnnnnnnnn     180

nnnnnnnnnn nnnnnnnnnn nnnnnnnnnn nnnnnnnnnn nnnnnnnnnn nnnnnnnnnn     240

nnnnnnnnnn nnnnnnnnnn nnnnnnnnnn nnnnnnnnnn nnnnnnnnnn nnnnnnnnnn     300

nnnnnnnnnn nnnnnnnnnn nnnnnnnnnn nnnnnnnnnn nnnnnnnnnn nnnnnnnnnn     360

nnnnnnnnnn nnnnnnnnnn nnnnnnn                                          387


<210> 11
<211> 396
<212> RNA
<213> Artificial Sequence

<220>
<223> Description of Artificial Sequence: Synthetic
      polynucleotide


<220>
<221> modified_base
<222> (1)..(20)
<223> a, c, u or g and this region may encompass 17-20 nucleotides
      wherein some positions may be absent

<220>
<221> modified_base
<222> (97)..(396)
<223> a, c, u or g and this region may encompass 0-300 nucleotides
      wherein some positions may be absent

<400> 11
nnnnnnnnnn nnnnnnnnnn guuuuagagc uagaaauagc aaguuaaaau aaggcuaguc      60

cguuaucaac uugaaaaagu ggcaccgagu cggugcnnnn nnnnnnnnnn nnnnnnnnnn     120

nnnnnnnnnn nnnnnnnnnn nnnnnnnnnn nnnnnnnnnn nnnnnnnnnn nnnnnnnnnn     180

nnnnnnnnnn nnnnnnnnnn nnnnnnnnnn nnnnnnnnnn nnnnnnnnnn nnnnnnnnnn     240

nnnnnnnnnn nnnnnnnnnn nnnnnnnnnn nnnnnnnnnn nnnnnnnnnn nnnnnnnnnn     300

nnnnnnnnnn nnnnnnnnnn nnnnnnnnnn nnnnnnnnnn nnnnnnnnnn nnnnnnnnnn 360

nnnnnnnnnn nnnnnnnnnn nnnnnnnnnn nnnnnn 396

<210> 12
<211> 96
<212> RNA
<213> Artificial Sequence

<220>
<223> Description of Artificial Sequence: Synthetic
oligonucleotide

<220>
<221> modified_base
<222> (1)..(20)
<223> a, c, u or g and this region may encompass 17-20 nucleotides
wherein some positions may be absent

<400> 12
nnnnnnnnnn nnnnnnnnnn guuuaagagc uagaaauagc aaguuuaaau aaggcuaguc 60

cguuaucaac uugaaaaagu ggcaccgagu cggugc 96

<210> 13
<211> 106
<212> RNA
<213> Artificial Sequence

<220>
<223> Description of Artificial Sequence: Synthetic
polynucleotide

<220>
<221> modified_base
<222> (1)..(20)
<223> a, c, u or g and this region may encompass 17-20 nucleotides
wherein some positions may be absent

<400> 13
nnnnnnnnnn nnnnnnnnnn guuuuagagc uaugcuggaa acagcauagc aaguuuaaau 60

aaggcuaguc cguuaucaac uugaaaaagu ggcaccgagu cggugc 106

<210> 14
<211> 106
<212> RNA
<213> Artificial Sequence

<220>
<223> Description of Artificial Sequence: Synthetic
polynucleotide

<220>
<221> modified_base
<222> (1)..(20)
<223> a, c, u or g and this region may encompass 17-20 nucleotides

wherein some positions may be absent

<400> 14
nnnnnnnnnn nnnnnnnnnn guuuaagagc uaugcuggaa acagcauagc aaguuuaaau 60

aaggcuaguc cguuaucaac uugaaaaagu ggcaccgagu cggugc 106


<210> 15
<211> 36
<212> DNA
<213> Artificial Sequence

<220>
<223> Description of Artificial Sequence: Synthetic
      oligonucleotide

<220>
<223> Description of Combined DNA/RNA Molecule: Synthetic
      oligonucleotide


<220>
<221> modified_base
<222> (1)..(20)
<223> a, c, u or g and this region may encompass 17-20 nucleotides
      wherein some positions may be absent

<400> 15
nnnnnnnnnn nnnnnnnnnn gttttagagc tagaaa 36


<210> 16
<211> 60
<212> DNA
<213> Artificial Sequence

<220>
<223> Description of Artificial Sequence: Synthetic
      oligonucleotide

<400> 16
tagcaagtta aaataaggct agtccgttat caacttgaaa aagtggcacc gagtcggtgc 60


<210> 17
<211> 5
<212> PRT
<213> Artificial Sequence

<220>
<223> Description of Artificial Sequence: Synthetic
      peptide

<400> 17
Gly Gly Gly Gly Ser
1               5


<210> 18
<211> 13
<212> PRT
<213> Artificial Sequence

<220>
<223> Description of Artificial Sequence: Synthetic
      peptide

<400> 18
Asp Ala Tyr Pro Tyr Asp Val Pro Asp Tyr Ala Ser Leu
1               5                   10

<210> 19
<211> 17
<212> PRT
<213> Artificial Sequence

<220>
<223> Description of Artificial Sequence: Synthetic
      peptide

<400> 19
Pro Lys Lys Lys Arg Lys Val Glu Asp Pro Lys Lys Lys Arg Lys Val
1               5                   10                  15

Asp

<210> 20
<211> 1401
<212> PRT
<213> Artificial Sequence

<220>
<223> Description of Artificial Sequence: Synthetic
      polypeptide

<400> 20
Met Asp Lys Lys Tyr Ser Ile Gly Leu Asp Ile Gly Thr Asn Ser Val
1               5                   10                  15

Gly Trp Ala Val Ile Thr Asp Glu Tyr Lys Val Pro Ser Lys Lys Phe
            20                  25                  30

Lys Val Leu Gly Asn Thr Asp Arg His Ser Ile Lys Lys Asn Leu Ile
        35                  40                  45

Gly Ala Leu Leu Phe Asp Ser Gly Glu Thr Ala Glu Ala Thr Arg Leu
    50                  55                  60

Lys Arg Thr Ala Arg Arg Arg Tyr Thr Arg Arg Lys Asn Arg Ile Cys
65                  70                  75                  80

Tyr Leu Gln Glu Ile Phe Ser Asn Glu Met Ala Lys Val Asp Asp Ser
                85                  90                  95

Phe Phe His Arg Leu Glu Glu Ser Phe Leu Val Glu Glu Asp Lys Lys

            100                 105                 110

His Glu Arg His Pro Ile Phe Gly Asn Ile Val Asp Glu Val Ala Tyr
        115                 120                 125

His Glu Lys Tyr Pro Thr Ile Tyr His Leu Arg Lys Lys Leu Val Asp
    130                 135                 140

Ser Thr Asp Lys Ala Asp Leu Arg Leu Ile Tyr Leu Ala Leu Ala His
145                 150                 155                 160

Met Ile Lys Phe Arg Gly His Phe Leu Ile Glu Gly Asp Leu Asn Pro
                165                 170                 175

Asp Asn Ser Asp Val Asp Lys Leu Phe Ile Gln Leu Val Gln Thr Tyr
            180                 185                 190

Asn Gln Leu Phe Glu Glu Asn Pro Ile Asn Ala Ser Gly Val Asp Ala
        195                 200                 205

Lys Ala Ile Leu Ser Ala Arg Leu Ser Lys Ser Arg Arg Leu Glu Asn
    210                 215                 220

Leu Ile Ala Gln Leu Pro Gly Glu Lys Lys Asn Gly Leu Phe Gly Asn
225                 230                 235                 240

Leu Ile Ala Leu Ser Leu Gly Leu Thr Pro Asn Phe Lys Ser Asn Phe
                245                 250                 255

Asp Leu Ala Glu Asp Ala Lys Leu Gln Leu Ser Lys Asp Thr Tyr Asp
            260                 265                 270

Asp Asp Leu Asp Asn Leu Leu Ala Gln Ile Gly Asp Gln Tyr Ala Asp
        275                 280                 285

Leu Phe Leu Ala Ala Lys Asn Leu Ser Asp Ala Ile Leu Leu Ser Asp
    290                 295                 300

Ile Leu Arg Val Asn Thr Glu Ile Thr Lys Ala Pro Leu Ser Ala Ser
305                 310                 315                 320

Met Ile Lys Arg Tyr Asp Glu His His Gln Asp Leu Thr Leu Leu Lys
                325                 330                 335

Ala Leu Val Arg Gln Gln Leu Pro Glu Lys Tyr Lys Glu Ile Phe Phe
            340                 345                 350

Asp Gln Ser Lys Asn Gly Tyr Ala Gly Tyr Ile Asp Gly Gly Ala Ser
        355                 360                 365

Gln Glu Glu Phe Tyr Lys Phe Ile Lys Pro Ile Leu Glu Lys Met Asp
    370                 375                 380

Gly Thr Glu Glu Leu Leu Val Lys Leu Asn Arg Glu Asp Leu Leu Arg
385                 390                 395                 400

Lys Gln Arg Thr Phe Asp Asn Gly Ser Ile Pro His Gln Ile His Leu
                405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
                485                 490                 495

Asn Phe Asp Lys Asn Leu Pro Asn Glu Lys Val Leu Pro Lys His Ser
            500                 505                 510

Leu Leu Tyr Glu Tyr Phe Thr Val Tyr Asn Glu Leu Thr Lys Val Lys
        515                 520                 525

Tyr Val Thr Glu Gly Met Arg Lys Pro Ala Phe Leu Ser Gly Glu Gln
    530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
                565                 570                 575

Ser Val Glu Ile Ser Gly Val Glu Asp Arg Phe Asn Ala Ser Leu Gly
            580                 585                 590

Thr Tyr His Asp Leu Leu Lys Ile Ile Lys Asp Lys Asp Phe Leu Asp
        595                 600                 605

Asn Glu Glu Asn Glu Asp Ile Leu Glu Asp Ile Val Leu Thr Leu Thr
    610                 615                 620

Leu Phe Glu Asp Arg Glu Met Ile Glu Glu Arg Leu Lys Thr Tyr Ala
625                 630                 635                 640

His Leu Phe Asp Asp Lys Val Met Lys Gln Leu Lys Arg Arg Arg Tyr
                645                 650                 655

Thr Gly Trp Gly Arg Leu Ser Arg Lys Leu Ile Asn Gly Ile Arg Asp
            660                 665                 670

Lys Gln Ser Gly Lys Thr Ile Leu Asp Phe Leu Lys Ser Asp Gly Phe
        675                 680                 685

Ala Asn Arg Asn Phe Met Gln Leu Ile His Asp Asp Ser Leu Thr Phe
    690                 695                 700

Lys Glu Asp Ile Gln Lys Ala Gln Val Ser Gly Gln Gly Asp Ser Leu
705                 710                 715                 720

His Glu His Ile Ala Asn Leu Ala Gly Ser Pro Ala Ile Lys Lys Gly
                725                 730                 735

Ile Leu Gln Thr Val Lys Val Val Asp Glu Leu Val Lys Val Met Gly
            740                 745                 750

Arg His Lys Pro Glu Asn Ile Val Ile Glu Met Ala Arg Glu Asn Gln
        755                 760                 765

Thr Thr Gln Lys Gly Gln Lys Asn Ser Arg Glu Arg Met Lys Arg Ile
    770                 775                 780

Glu Glu Gly Ile Lys Glu Leu Gly Ser Gln Ile Leu Lys Glu His Pro
785                 790                 795                 800

Val Glu Asn Thr Gln Leu Gln Asn Glu Lys Leu Tyr Leu Tyr Tyr Leu
                805                 810                 815

Gln Asn Gly Arg Asp Met Tyr Val Asp Gln Glu Leu Asp Ile Asn Arg
            820                 825                 830

Leu Ser Asp Tyr Asp Val Asp His Ile Val Pro Gln Ser Phe Leu Lys
        835                 840                 845

Asp Asp Ser Ile Asp Asn Lys Val Leu Thr Arg Ser Asp Lys Asn Arg
    850                 855                 860

Gly Lys Ser Asp Asn Val Pro Ser Glu Glu Val Val Lys Lys Met Lys
865                 870                 875                 880

Asn Tyr Trp Arg Gln Leu Leu Asn Ala Lys Leu Ile Thr Gln Arg Lys
                885                 890                 895

Phe Asp Asn Leu Thr Lys Ala Glu Arg Gly Gly Leu Ser Glu Leu Asp
            900                 905                 910

Lys Ala Gly Phe Ile Lys Arg Gln Leu Val Glu Thr Arg Gln Ile Thr
        915                 920                 925

Lys His Val Ala Gln Ile Leu Asp Ser Arg Met Asn Thr Lys Tyr Asp
    930                 935                 940

Glu Asn Asp Lys Leu Ile Arg Glu Val Lys Val Ile Thr Leu Lys Ser
945                 950                 955                 960

Lys Leu Val Ser Asp Phe Arg Lys Asp Phe Gln Phe Tyr Lys Val Arg
                965                 970                 975

Glu Ile Asn Asn Tyr His His Ala His Asp Ala Tyr Leu Asn Ala Val
            980                 985                 990

Val Gly Thr Ala Leu Ile Lys Lys  Tyr Pro Lys Leu Glu  Ser Glu Phe
        995                 1000                1005

Val Tyr  Gly Asp Tyr Lys Val  Tyr Asp Val Arg Lys  Met Ile Ala
    1010                1015                1020

Lys Ser  Glu Gln Glu Ile Gly  Lys Ala Thr Ala Lys  Tyr Phe Phe
    1025                1030                1035

Tyr Ser  Asn Ile Met Asn Phe  Phe Lys Thr Glu Ile  Thr Leu Ala
    1040                1045                1050

Asn Gly  Glu Ile Arg Lys Arg  Pro Leu Ile Glu Thr  Asn Gly Glu
    1055                1060                1065

Thr Gly  Glu Ile Val Trp Asp  Lys Gly Arg Asp Phe  Ala Thr Val
    1070                1075                1080

Arg Lys  Val Leu Ser Met Pro  Gln Val Asn Ile Val  Lys Lys Thr
    1085                1090                1095

Glu Val  Gln Thr Gly Gly Phe  Ser Lys Glu Ser Ile  Leu Pro Lys

1100                1105                1110

Arg Asn Ser Asp Lys Leu Ile Ala Arg Lys Lys Asp Trp Asp Pro
    1115                1120                1125

Lys Lys Tyr Gly Gly Phe Asp Ser Pro Thr Val Ala Tyr Ser Val
    1130                1135                1140

Leu Val Val Ala Lys Val Glu Lys Gly Lys Ser Lys Lys Leu Lys
    1145                1150                1155

Ser Val Lys Glu Leu Leu Gly Ile Thr Ile Met Glu Arg Ser Ser
    1160                1165                1170

Phe Glu Lys Asn Pro Ile Asp Phe Leu Glu Ala Lys Gly Tyr Lys
    1175                1180                1185

Glu Val Lys Lys Asp Leu Ile Ile Lys Leu Pro Lys Tyr Ser Leu
    1190                1195                1200

Phe Glu Leu Glu Asn Gly Arg Lys Arg Met Leu Ala Ser Ala Gly
    1205                1210                1215

Glu Leu Gln Lys Gly Asn Glu Leu Ala Leu Pro Ser Lys Tyr Val
    1220                1225                1230

Asn Phe Leu Tyr Leu Ala Ser His Tyr Glu Lys Leu Lys Gly Ser
    1235                1240                1245

Pro Glu Asp Asn Glu Gln Lys Gln Leu Phe Val Glu Gln His Lys
    1250                1255                1260

His Tyr Leu Asp Glu Ile Ile Glu Gln Ile Ser Glu Phe Ser Lys
    1265                1270                1275

Arg Val Ile Leu Ala Asp Ala Asn Leu Asp Lys Val Leu Ser Ala
    1280                1285                1290

Tyr Asn Lys His Arg Asp Lys Pro Ile Arg Glu Gln Ala Glu Asn
    1295                1300                1305

Ile Ile His Leu Phe Thr Leu Thr Asn Leu Gly Ala Pro Ala Ala
    1310                1315                1320

Phe Lys Tyr Phe Asp Thr Thr Ile Asp Arg Lys Arg Tyr Thr Ser
    1325                1330                1335

Thr Lys Glu Val Leu Asp Ala Thr Leu Ile His Gln Ser Ile Thr
    1340                1345                1350

Gly Leu Tyr Glu Thr Arg Ile Asp Leu Ser Gln Leu Gly Gly Asp
    1355                1360                1365

Ala Tyr Pro Tyr Asp Val Pro Asp Tyr Ala Ser Leu Gly Ser Gly
    1370                1375                1380

Ser Pro Lys Lys Lys Arg Lys Val Glu Asp Pro Lys Lys Lys Arg
    1385                1390                1395

Lys Val Asp
    1400

<210> 21
<211> 1368
<212> PRT
<213> Artificial Sequence

<220>
<223> Description of Artificial Sequence: Synthetic
      polypeptide

<400> 21
Met Asp Lys Lys Tyr Ser Ile Gly Leu Ala Ile Gly Thr Asn Ser Val
1               5                   10                  15

Gly Trp Ala Val Ile Thr Asp Glu Tyr Lys Val Pro Ser Lys Lys Phe
            20                  25                  30

Lys Val Leu Gly Asn Thr Asp Arg His Ser Ile Lys Lys Asn Leu Ile
        35                  40                  45

Gly Ala Leu Leu Phe Asp Ser Gly Glu Thr Ala Glu Ala Thr Arg Leu
    50                  55                  60

Lys Arg Thr Ala Arg Arg Arg Tyr Thr Arg Arg Lys Asn Arg Ile Cys
65                  70                  75                  80

Tyr Leu Gln Glu Ile Phe Ser Asn Glu Met Ala Lys Val Asp Asp Ser
                85                  90                  95

Phe Phe His Arg Leu Glu Glu Ser Phe Leu Val Glu Glu Asp Lys Lys
            100                 105                 110

His Glu Arg His Pro Ile Phe Gly Asn Ile Val Asp Glu Val Ala Tyr
        115                 120                 125

His Glu Lys Tyr Pro Thr Ile Tyr His Leu Arg Lys Lys Leu Val Asp

130                 135                 140

Ser Thr Asp Lys Ala Asp Leu Arg Leu Ile Tyr Leu Ala Leu Ala His
145                 150                 155                 160

Met Ile Lys Phe Arg Gly His Phe Leu Ile Glu Gly Asp Leu Asn Pro
                165                 170                 175

Asp Asn Ser Asp Val Asp Lys Leu Phe Ile Gln Leu Val Gln Thr Tyr
            180                 185                 190

Asn Gln Leu Phe Glu Glu Asn Pro Ile Asn Ala Ser Gly Val Asp Ala
        195                 200                 205

Lys Ala Ile Leu Ser Ala Arg Leu Ser Lys Ser Arg Arg Leu Glu Asn
    210                 215                 220

Leu Ile Ala Gln Leu Pro Gly Glu Lys Lys Asn Gly Leu Phe Gly Asn
225                 230                 235                 240

Leu Ile Ala Leu Ser Leu Gly Leu Thr Pro Asn Phe Lys Ser Asn Phe
                245                 250                 255

Asp Leu Ala Glu Asp Ala Lys Leu Gln Leu Ser Lys Asp Thr Tyr Asp
            260                 265                 270

Asp Asp Leu Asp Asn Leu Leu Ala Gln Ile Gly Asp Gln Tyr Ala Asp
        275                 280                 285

Leu Phe Leu Ala Ala Lys Asn Leu Ser Asp Ala Ile Leu Leu Ser Asp
    290                 295                 300

Ile Leu Arg Val Asn Thr Glu Ile Thr Lys Ala Pro Leu Ser Ala Ser
305                 310                 315                 320

Met Ile Lys Arg Tyr Asp Glu His His Gln Asp Leu Thr Leu Leu Lys
                325                 330                 335

Ala Leu Val Arg Gln Gln Leu Pro Glu Lys Tyr Lys Glu Ile Phe Phe
            340                 345                 350

Asp Gln Ser Lys Asn Gly Tyr Ala Gly Tyr Ile Asp Gly Gly Ala Ser
        355                 360                 365

Gln Glu Glu Phe Tyr Lys Phe Ile Lys Pro Ile Leu Glu Lys Met Asp
    370                 375                 380

Gly Thr Glu Glu Leu Leu Val Lys Leu Asn Arg Glu Asp Leu Leu Arg
385                 390                 395                 400

Lys Gln Arg Thr Phe Asp Asn Gly Ser Ile Pro His Gln Ile His Leu
                405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
                485                 490                 495

Asn Phe Asp Lys Asn Leu Pro Asn Glu Lys Val Leu Pro Lys His Ser
            500                 505                 510

Leu Leu Tyr Glu Tyr Phe Thr Val Tyr Asn Glu Leu Thr Lys Val Lys
        515                 520                 525

Tyr Val Thr Glu Gly Met Arg Lys Pro Ala Phe Leu Ser Gly Glu Gln
    530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
                565                 570                 575

Ser Val Glu Ile Ser Gly Val Glu Asp Arg Phe Asn Ala Ser Leu Gly
            580                 585                 590

Thr Tyr His Asp Leu Leu Lys Ile Ile Lys Asp Lys Asp Phe Leu Asp
        595                 600                 605

Asn Glu Glu Asn Glu Asp Ile Leu Glu Asp Ile Val Leu Thr Leu Thr
    610                 615                 620

Leu Phe Glu Asp Arg Glu Met Ile Glu Glu Arg Leu Lys Thr Tyr Ala
625                 630                 635                 640

His Leu Phe Asp Asp Lys Val Met Lys Gln Leu Lys Arg Arg Arg Tyr
                645                 650                 655

Thr Gly Trp Gly Arg Leu Ser Arg Lys Leu Ile Asn Gly Ile Arg Asp
            660                 665                 670

Lys Gln Ser Gly Lys Thr Ile Leu Asp Phe Leu Lys Ser Asp Gly Phe
        675                 680                 685

Ala Asn Arg Asn Phe Met Gln Leu Ile His Asp Asp Ser Leu Thr Phe
    690                 695                 700

Lys Glu Asp Ile Gln Lys Ala Gln Val Ser Gly Gln Gly Asp Ser Leu
705                 710                 715                 720

His Glu His Ile Ala Asn Leu Ala Gly Ser Pro Ala Ile Lys Lys Gly
                725                 730                 735

Ile Leu Gln Thr Val Lys Val Val Asp Glu Leu Val Lys Val Met Gly
            740                 745                 750

Arg His Lys Pro Glu Asn Ile Val Ile Glu Met Ala Arg Glu Asn Gln
        755                 760                 765

Thr Thr Gln Lys Gly Gln Lys Asn Ser Arg Glu Arg Met Lys Arg Ile
    770                 775                 780

Glu Glu Gly Ile Lys Glu Leu Gly Ser Gln Ile Leu Lys Glu His Pro
785                 790                 795                 800

Val Glu Asn Thr Gln Leu Gln Asn Glu Lys Leu Tyr Leu Tyr Tyr Leu
                805                 810                 815

Gln Asn Gly Arg Asp Met Tyr Val Asp Gln Glu Leu Asp Ile Asn Arg
            820                 825                 830

Leu Ser Asp Tyr Asp Val Asp Ala Ile Val Pro Gln Ser Phe Leu Lys
        835                 840                 845

Asp Asp Ser Ile Asp Asn Lys Val Leu Thr Arg Ser Asp Lys Asn Arg
    850                 855                 860

Gly Lys Ser Asp Asn Val Pro Ser Glu Glu Val Val Lys Lys Met Lys
865                 870                 875                 880

Asn Tyr Trp Arg Gln Leu Leu Asn Ala Lys Leu Ile Thr Gln Arg Lys
                885                 890                 895

Phe Asp Asn Leu Thr Lys Ala Glu Arg Gly Gly Leu Ser Glu Leu Asp
            900                 905                 910

Lys Ala Gly Phe Ile Lys Arg Gln Leu Val Glu Thr Arg Gln Ile Thr
        915                 920                 925

Lys His Val Ala Gln Ile Leu Asp Ser Arg Met Asn Thr Lys Tyr Asp
    930                 935                 940

Glu Asn Asp Lys Leu Ile Arg Glu Val Lys Val Ile Thr Leu Lys Ser
945                 950                 955                 960

Lys Leu Val Ser Asp Phe Arg Lys Asp Phe Gln Phe Tyr Lys Val Arg
                965                 970                 975

Glu Ile Asn Asn Tyr His His Ala His Asp Ala Tyr Leu Asn Ala Val
            980                 985                 990

Val Gly Thr Ala Leu Ile Lys Lys Tyr Pro Lys Leu Glu Ser Glu Phe
        995                 1000                1005

Val Tyr Gly Asp Tyr Lys Val Tyr Asp Val Arg Lys Met Ile Ala
    1010                1015                1020

Lys Ser Glu Gln Glu Ile Gly Lys Ala Thr Ala Lys Tyr Phe Phe
    1025                1030                1035

Tyr Ser Asn Ile Met Asn Phe Phe Lys Thr Glu Ile Thr Leu Ala
    1040                1045                1050

Asn Gly Glu Ile Arg Lys Arg Pro Leu Ile Glu Thr Asn Gly Glu
    1055                1060                1065

Thr Gly Glu Ile Val Trp Asp Lys Gly Arg Asp Phe Ala Thr Val
    1070                1075                1080

Arg Lys Val Leu Ser Met Pro Gln Val Asn Ile Val Lys Lys Thr
    1085                1090                1095

Glu Val Gln Thr Gly Gly Phe Ser Lys Glu Ser Ile Leu Pro Lys
    1100                1105                1110

Arg Asn Ser Asp Lys Leu Ile Ala Arg Lys Lys Asp Trp Asp Pro
    1115                1120                1125

Lys Lys Tyr Gly Gly Phe Asp Ser Pro Thr Val Ala Tyr Ser Val

1130                1135                1140

Leu Val Val Ala Lys Val Glu Lys Gly Lys Ser Lys Lys Leu Lys
    1145                1150                1155

Ser Val Lys Glu Leu Leu Gly Ile Thr Ile Met Glu Arg Ser Ser
    1160                1165                1170

Phe Glu Lys Asn Pro Ile Asp Phe Leu Glu Ala Lys Gly Tyr Lys
    1175                1180                1185

Glu Val Lys Lys Asp Leu Ile Ile Lys Leu Pro Lys Tyr Ser Leu
    1190                1195                1200

Phe Glu Leu Glu Asn Gly Arg Lys Arg Met Leu Ala Ser Ala Gly
    1205                1210                1215

Glu Leu Gln Lys Gly Asn Glu Leu Ala Leu Pro Ser Lys Tyr Val
    1220                1225                1230

Asn Phe Leu Tyr Leu Ala Ser His Tyr Glu Lys Leu Lys Gly Ser
    1235                1240                1245

Pro Glu Asp Asn Glu Gln Lys Gln Leu Phe Val Glu Gln His Lys
    1250                1255                1260

His Tyr Leu Asp Glu Ile Ile Glu Gln Ile Ser Glu Phe Ser Lys
    1265                1270                1275

Arg Val Ile Leu Ala Asp Ala Asn Leu Asp Lys Val Leu Ser Ala
    1280                1285                1290

Tyr Asn Lys His Arg Asp Lys Pro Ile Arg Glu Gln Ala Glu Asn
    1295                1300                1305

Ile Ile His Leu Phe Thr Leu Thr Asn Leu Gly Ala Pro Ala Ala
    1310                1315                1320

Phe Lys Tyr Phe Asp Thr Thr Ile Asp Arg Lys Arg Tyr Thr Ser
    1325                1330                1335

Thr Lys Glu Val Leu Asp Ala Thr Leu Ile His Gln Ser Ile Thr
    1340                1345                1350

Gly Leu Tyr Glu Thr Arg Ile Asp Leu Ser Gln Leu Gly Gly Asp
    1355                1360                1365

<210> 22
<211> 4
<212> PRT
<213> Artificial Sequence

<220>
<223> Description of Artificial Sequence: Synthetic
      peptide

<400> 22
Gly Gly Ser Gly
1


<210> 23
<211> 12
<212> PRT
<213> Artificial Sequence

<220>
<223> Description of Artificial Sequence: Synthetic
      peptide

<400> 23
Ser Gly Ser Glu Thr Pro Gly Thr Ser Glu Ser Ala
1               5                   10


<210> 24
<211> 16
<212> PRT
<213> Artificial Sequence

<220>
<223> Description of Artificial Sequence: Synthetic
      peptide

<400> 24
Ser Gly Ser Glu Thr Pro Gly Thr Ser Glu Ser Ala Thr Pro Glu Ser
1               5                   10                  15


<210> 25
<211> 21
<212> PRT
<213> Artificial Sequence

<220>
<223> Description of Artificial Sequence: Synthetic
      peptide

<400> 25
Ser Gly Ser Glu Thr Pro Gly Thr Ser Glu Ser Ala Thr Pro Glu Gly
1               5                   10                  15


Gly Ser Gly Gly Ser
            20


<210> 26
<211> 24
<212> DNA

<213> Artificial Sequence

<220>
<223> Description of Artificial Sequence: Synthetic
      oligonucleotide

<400> 26
gccgaggtga agttcgaggg cgac                                              24


<210> 27
<211> 23
<212> DNA
<213> Artificial Sequence

<220>
<223> Description of Artificial Sequence: Synthetic
      oligonucleotide

<400> 27
cctacggcgt gcagtgcttc agc                                               23


<210> 28
<211> 332
<212> DNA
<213> Artificial Sequence

<220>
<223> Description of Artificial Sequence: Synthetic
      polynucleotide

<400> 28
acgtaaacgg ccacaagttc agcgtgtccg gcgagggcga gggcgatgcc acctacggca       60

agctgaccct gaagttcatc tgcaccaccg gcaagctgcc cgtgccctgg cccaccctcg      120

tgaccaccct gacctacggc gtgcagtgct tcagccgcta ccccgaccac atgaagcagc      180

acgacttctt caagtccgcc atgcccgaag gctacgtcca ggagcgcacc atcttcttca      240

aggacgacgg caactacaag acccgcgccg aggtgaagtt cgagggcgac accctggtga      300

accgcatcga gctgaagggc atcgacttca ag                                    332


<210> 29
<211> 295
<212> DNA
<213> Artificial Sequence

<220>
<223> Description of Artificial Sequence: Synthetic
      polynucleotide

<400> 29
gtccggcgag ggcgagggcg atgccaccta cggcaagctg accctgaagt tcatctgcac       60

caccggcaag ctgcccgtgc cctggcccac cctcgtgacc accctgacct acggcgtgca      120

gtgcttcagc cgctaccccg accacatgaa gcagcacgac ttcttcaagt ccgccatgcc      180

cgaaggctac gtccaggagc gcaccatctt cttcaaggac gacggcaact acaagacccg      240

cgtcgagggc gacaccctgg tgaaccgcat cgagctgaag ggcatcgact tcaag 295

<210> 30
<211> 158
<212> DNA
<213> Artificial Sequence

<220>
<223> Description of Artificial Sequence: Synthetic
      polynucleotide

<400> 30
tccggcgagg gcgagggcga tgccacctac ggcaagctga ccctgaagtt catctgcacc 60

accggcaagc tgcccgtgcc ctggcccacc ctcgtgacca ccctgacgag ggcgacaccc 120

tggtgaaccg catcgagctg aagggcatcg acttcaag 158

<210> 31
<211> 285
<212> DNA
<213> Artificial Sequence

<220>
<223> Description of Artificial Sequence: Synthetic
      polynucleotide

<400> 31
tccggcgagg gcgagggcga tgccacctac ggcaagctga ccctgaagtt catctgcacc 60

accggcaagc tgcccgtgcc ctggcccacc ctcgtgaaca ccctgaccta cgtgcagtgc 120

ttcagccgct accccgacca catgaagcag cacgacttct tcaagtccgc catgcccgaa 180

ggctacgtcc aggagcgcac catcttcttc aaggacgacg gcaactacaa gttcgagggc 240

gacaccctgg tgaaccgcat cgagctgaag ggcatcgact tcaag 285

<210> 32
<211> 301
<212> DNA
<213> Artificial Sequence

<220>
<223> Description of Artificial Sequence: Synthetic
      polynucleotide

<400> 32
ccggcgaggg cgagggcgat gccacctacg gcaagctgac cctgaagttc atctgcacca 60

ccggcaagct gcccgtgccc tggcccaccc tcgtgaccac cctgacctac gtgcagtgct 120

tcagccgcta ccccgaccac atgaagcagc acgacttctt caagtccgcc atgcccgaag 180

gctacgtcca ggagcgcacc atcttcttca aggacgacgg caactacaag acccgcgccg 240

aggtgaagtt cgagggcgac accctggtga accgcatcga gctgaagggc atcgacttca 300

a 301

<210> 33
<211> 156
<212> DNA
<213> Artificial Sequence

<220>
<223> Description of Artificial Sequence: Synthetic
      polynucleotide

<400> 33
tccggcgagg gcgagggcga tgccacctac ggcaagctga ccctgaagtt catctgcacc      60

accggcaagc tgcccgtgcc ctggcccacc ctcgtgacca ccctgaccta cggacaccct     120

ggtgaaccgc atcgagctga agggcatcga cttcaa                                156


<210> 34
<211> 197
<212> DNA
<213> Artificial Sequence

<220>
<223> Description of Artificial Sequence: Synthetic
      polynucleotide

<400> 34
gtaaacggcc acaagttcag cgtgcagtgc ttcagccgct accccgacca catgaagcag      60

cacgacttct tcaagtccgc catgcccgaa ggctacgtcc aggagcgcac catcttcttc     120

aaggacgacg gcaactacaa gacggcaact acaagttcga gggcgacacc ctggtgaacc     180

gcatcgagct gaagggc                                                     197


<210> 35
<211> 283
<212> DNA
<213> Artificial Sequence

<220>
<223> Description of Artificial Sequence: Synthetic
      polynucleotide

<400> 35
ccacaagttc agcgtgtccg gcgagggcga gggcgatgcc acctacggca agctgaccct      60

gaagttcatc tgcaccaccg gcaagctgcc cgtgccctgg cccaccctcg tgaccaccct     120

gacctacggc gtgcagtgct tcagccgcta ccccgaccac atgaagcagc acgacttctt     180

caagtccgcc atgcccgaag gctacgtcca ggagcgcacc atcttcttca aggacgacgg     240

caactacaag acaccgcatc gagctgaagg gcatcgactt caa                        283


<210> 36
<211> 175
<212> DNA
<213> Artificial Sequence

<220>
<223> Description of Artificial Sequence: Synthetic
      polynucleotide

<400> 36
ggccacaagt tcagcgtgtc cggcgagggc gagggcgatg ccacctacgg caagctgacc      60

ctgaagttca tctgcaccac cggcaccatc aaggacgacg gcaactacaa gacccgcgcc     120

gaagttcgag ggcgacaccc tggtgaaccg catcgagctg aagggcatcg acttc          175


<210> 37
<211> 291
<212> DNA
<213> Artificial Sequence

<220>
<223> Description of Artificial Sequence: Synthetic
      polynucleotide

<400> 37
taaacggcca caagttcagc gtgtccggcg agggcgaggg cgatgccacc tacggcaagc      60

tgaccctgaa gttcatctgc accaccggca agctgcccgt gccctggccc accctcgtga     120

ccaccctgac ctacggcgtg cagtgcttca gccgctaccc cgaccacatg aagcagcacg     180

acttcttcaa gtccgccatg cccgaaggct acgtccagga gcgcaccatc ttcttcaagg     240

acgacggcaa ctacaagacc tggtgaaccg catcgagctg aagggcatcg a              291


<210> 38
<211> 314
<212> DNA
<213> Artificial Sequence

<220>
<223> Description of Artificial Sequence: Synthetic
      polynucleotide

<400> 38
cacaagttca gcgtgtccgg cgagggcgag ggcgatgcca cctacggcaa gctgaccctg      60

aagttcatct gcaccaccgg caagctgccc gtgccctggc ccaccctcgt gaccaccctg     120

acctacggcg tgcagtgctt cagccgctac cccgaccaca tgaagcagca cgacttcttc     180

aagtccgcca tgcccgaagg ctacgtccag gagcgcacca tcttcttcaa ggacgacggc     240

aactacaaga cccgcgccga ggttcgaggg cgacaccctg gtgaaccgca tcgagctgaa     300

gggcatcgac ttca                                                       314


<210> 39
<211> 316
<212> DNA
<213> Artificial Sequence

<220>

<223> Description of Artificial Sequence: Synthetic
      polynucleotide

<400> 39
taaacggcca caagttcagc gtgtccggcg agggcgaggg cgatgccacc tacggcaagc      60

tgaccctgaa gttcatctgc accaccggca agctgcccgt gccctggccc accctcgtga     120

ccaccctgac ctacggcgtg cagtgcttca gccgctaccc cgaccacatg aagcagcacg     180

acttcttcaa gtccgccatg cccgaaggct acgtccagga gcgcaccatc ttcttcaagg     240

acgacggcaa ctacaagacc cgcgccgagg gcgacaccct ggtgaaccgc atcgagctga     300

agggcatcga cttcaa                                                     316


<210> 40
<211> 318
<212> DNA
<213> Artificial Sequence

<220>
<223> Description of Artificial Sequence: Synthetic
      polynucleotide

<400> 40
aaacggccac aagttcagcg tgtccggcga gggcgagggc gatgccacct acggcaagct      60

gaccctgaag ttcatctgca ccaccggcaa gctgcccgtg ccctggccca ccctcgtgac     120

caccctgacc tacgtgcagt gcttcagccg ctaccccgac cacatgaagc agcacgactt     180

cttcaagtcc gccatgcccg aaggctacgt ccaggagcgc accatcttct tcaaggacga     240

cggcaactac aagacccgcg ccgaggtgaa gttcgagggc gacaccctgg tgaaccgcat     300

cgagctgaag ggcatcga                                                   318


<210> 41
<211> 185
<212> DNA
<213> Artificial Sequence

<220>
<223> Description of Artificial Sequence: Synthetic
      polynucleotide

<400> 41
acggccacaa gttcagcgtg tccggcgagg gcgagggcga tgccacctac ggcaagctga      60

ccctgaagtt catctgcacc accggcaagc tgcccgtgcc ctggcccacc ctcgtgacca     120

ccctgaccta cgtgaagttc gagggcgaca ccctggtgaa ccgcatcgag ctgaagggca     180

tcgac                                                                 185


<210> 42
<211> 180
<212> DNA
<213> Artificial Sequence

<220>
<223> Description of Artificial Sequence: Synthetic
      polynucleotide

<400> 42
aaacggccac aagttcagcg tgtccggcga gggcgagggc gatgccacct acggcaagct       60

gaccctgaag ttcatctgca ccaccggcaa gctgcccgtg ccctggccca ccctcgtgac      120

caccctgacc tacgtgaagt tcgagggcga caccctggtg aaccgcatcg agctgaaggg      180


<210> 43
<211> 242
<212> DNA
<213> Artificial Sequence

<220>
<223> Description of Artificial Sequence: Synthetic
      polynucleotide


<220>
<221> modified_base
<222> (132)..(132)
<223> a, c, t, g, unknown or other

<220>
<221> modified_base
<222> (153)..(153)
<223> a, c, t, g, unknown or other

<220>
<221> modified_base
<222> (174)..(174)
<223> a, c, t, g, unknown or other

<400> 43
aaacggccac aagttcagcg tgtccggcga gggcgagggc gatgccacct acggcaagct       60

gaccctgaag ttcatctgca ccaccggcaa gctgcccgtg ccctggccca cccttccgcc      120

atgcccgaag gntacgtcca ggagcgcacc atnttcttca aggacgacgg caantacaag      180

acccgcgccg aagttcgagg gcgacaccct ggtgaaccgc atcgagctga agggcatcga      240

ct                                                                     242


<210> 44
<211> 175
<212> DNA
<213> Artificial Sequence

<220>
<223> Description of Artificial Sequence: Synthetic
      polynucleotide

<400> 44
caccctgacc tacggcgtgc agtgcttcag ccgctacccc gaccacatga agcagcacga       60

cttcttcaag tccgccatgc ccgaaggcta cgtccaggag cgcaccatct tcttcaagga      120

cgacggcaac tacaagaccc gcgccgaggt gaagttcgag ggcgacaccc tggtg          175

<210> 45
<211> 39
<212> DNA
<213> Artificial Sequence

<220>
<223> Description of Artificial Sequence: Synthetic
      oligonucleotide

<400> 45
caccctgacc tacgtgaagt tcgagggcga caccctggt                            39

<210> 46
<211> 161
<212> DNA
<213> Artificial Sequence

<220>
<223> Description of Artificial Sequence: Synthetic
      polynucleotide

<400> 46
caccctgacc tacggcgtgc agtgcttcag ccgctacccc gaccacatga agcagcacga      60

cttcttcaag tccgccatgc ccgaaggcta cgtccaggag cgcaccatct tcttcaagga     120

cgacggcaac tacaagaccc gcgccgaggg cgacaccctg g                         161

<210> 47
<211> 145
<212> DNA
<213> Artificial Sequence

<220>
<223> Description of Artificial Sequence: Synthetic
      polynucleotide

<400> 47
caccctgacc tcagccgcta ccccgaccac atgaagcagc acgacttctt caagtccgcc      60

atgcccgaag gctacgtcca ggagcgcacc atcttcttca aggacgacgg caactacaag     120

acccgcgccg aggcgacacc ctggt                                           145

<210> 48
<211> 153
<212> DNA
<213> Artificial Sequence

<220>
<223> Description of Artificial Sequence: Synthetic
      polynucleotide

<400> 48
ttattataca tcggagccct gccaaaaaat caatgtgaag caaatcgcag cccgcctcct      60

gcctccgctc tactcactgg tgttcatctt tggttttgtg ggcaacatgc tggtcatcct     120

catcctgata aactgcaaaa ggctgaagag cat                                  153

<210> 49
<211> 113
<212> DNA
<213> Artificial Sequence

<220>
<223> Description of Artificial Sequence: Synthetic
      polynucleotide

<400> 49
ttattataca tcggagccct gccaaaaaat caatgtgaag caaatcgcag cccgcctccg      60

ctctactcac tggtgttcat ctttggtttt gtgggcaaca tgctggtcat cct            113

<210> 50
<211> 70
<212> DNA
<213> Artificial Sequence

<220>
<223> Description of Artificial Sequence: Synthetic
      oligonucleotide

<400> 50
ttattataca tcggagccct gccaaaaaat caatgtgaag caaatcgcag cccgcatgct      60

ggtcatcctc                                                             70

<210> 51
<211> 62
<212> RNA
<213> Artificial Sequence

<220>
<223> Description of Artificial Sequence: Synthetic
      oligonucleotide

<220>
<221> modified_base
<222> (1)..(20)
<223> a, c, u or g and this region may encompass 17-20 nucleotides
      wherein some positions may be absent

<400> 51
nnnnnnnnnn nnnnnnnnnn guuuuagagc uagaaauagc aaguuaaaau aaggcuaguc      60

cg                                                                     62

<210> 52
<211> 54
<212> RNA
<213> Artificial Sequence

<220>

<223> Description of Artificial Sequence: Synthetic
      oligonucleotide

<220>
<221> modified_base
<222> (1)..(20)
<223> a, c, u or g and this region may encompass 17-20 nucleotides
      wherein some positions may be absent

<400> 52
nnnnnnnnnn nnnnnnnnnn guuuuagagc uagaaauagc aaguuaaaau aagg          54

<210> 53
<211> 28
<212> RNA
<213> Artificial Sequence

<220>
<223> Description of Artificial Sequence: Synthetic
      oligonucleotide

<400> 53
guucacugcc guauaggcag cuaagaaa                                        28

<210> 54
<211> 20
<212> RNA
<213> Artificial Sequence

<220>
<223> Description of Artificial Sequence: Synthetic
      oligonucleotide

<400> 54
guucacugcc guauaggcag                                                 20

## Description

### CROSS-REFERENCE TO RELATED APPLICATIONS

This application claims the benefit of Patent Applications 61/921,007, filed on December 26, 2013; 14/211,117, filed on March 14, 2014; PCT/US2014/029068, filed on March 14, 2014; PCT/US2014/028630, filed on March 14, 2014; PCT/US2014/035162, filed on March 23, 2014; PCT/US2014/029304, filed on March 14, 2014; and 61/930,782, filed on January 23, 2014. All of the foregoing are incorporated herein by reference.

### FEDERALLY SPONSORED RESEARCH OR DEVELOPMENT

This invention was made with Government support under Grant No. DP1 GM105378 awarded by the National Institutes of Health. The Government has certain rights in the invention.

### TECHNICAL FIELD

Described are methods and constructs for the multiplex expression of highly active CRISPR guide RNAs (gRNAs) from RNA Polymerase II and III promoters, optionally in mammalian cells.

### BACKGROUND

The Cas9 nuclease forms the basis of a programmable RNA-guided clustered, regularly interspaced, short palindromic repeats (CRISPR)/CRISPR-associated (Cas) system (Wiedenheft et al., Nature 482, 331-338 (2012); Horvath et al., Science 327, 167-170 (2010); Terns et al., Curr Opin Microbiol 14, 321-327 (2011)) that can be used to create site-specific breaks in target DNA sequences in vitro, in mammalian cells, and in living model organisms such as zebrafish (Wang et al., Cell 153, 910-918 (2013); Shen et al., Cell Res (2013); Dicarlo et al., Nucleic Acids Res (2013); Jiang et al., Nat Biotechnol 31, 233-239 (2013); Jinek et al., Elife 2, e00471 (2013); Hwang et al., Nat Biotechnol 31, 227-229 (2013); Cong et al., Science 339, 819-823 (2013); Mali et al., Science 339, 823-826 (2013c); Cho et al., Nat Biotechnol 31, 230-232 (2013); Gratz et al., Genetics 194(4):1029-35 (2013)). A short ~100 nt guide RNA (gRNA) complexes with Cas9 and directs the nuclease to a specific target DNA site; targeting is mediated by a sequence of at least 17-20 nucleotides (nts) at the 5' end of the gRNA, which are designed to be complementary to and interact via simple base pair complementarity between the first 17-20 nucleotides of an engineered gRNA and the complementary strand of a target genomic DNA sequence of interest that lies next to a protospacer adjacent motif (PAM), e.g., a PAM matching the sequence NGG or NAG (Shen et al., Cell Res (2013); Dicarlo et al., Nucleic Acids Res (2013); Jiang et al., Nat Biotechnol 31, 233-239 (2013); Jinek et al., Elife 2, e00471 (2013); Hwang et al., Nat Biotechnol 31, 227-229 (2013); Cong et al., Science 339, 819-823 (2013); Mali et al., Science 339, 823-826 (2013c); Cho et al., Nat Biotechnol 31, 230-232 (2013); Jinek et al., Science 337, 816-821 (2012)). gRNAs can also direct catalytically inactivated Cas9 proteins (known as dCas9, see Jinek et al., Science 337:816-821 (2012)) that are in turn fused to effector domains (e.g., a transcriptional activation domain) see, e.g., USSN 61/799,647, filed on March 15, 2013, and 61/838,148, filed on June 21, 2013, both of which are incorporated herein by reference. These latter systems enable RNA-guided recruitment of heterologous effector domains to genomic loci of interest.

### SUMMARY

The present invention is based, at least in part, on the discovery that Csy4, an endoribonuclease that recognizes a short RNA hairpin sequence, can be used to cleave out multiple functional gRNAs encoded on a single longer RNA transcript (produced from an RNA pol II or III promoter) in which the individual gRNAs are separated by Csy4 cleavage sites.

Thus in a first aspect the invention provides deoxyribonucleic acid (DNA) molecules comprising a plurality of sequences encoding guide RNAs (gRNAs), wherein each gRNA is flanked by at least one Csy4 cleavage sequence comprising or consisting of the sequence GTTCACTGCCGTATAGGCAG (SEQ ID NO:1) or GTTCACTGCCGTATAGGCAGCTAAGAAA (SEQ ID NO:2).

In some embodiments the DNA molecule is operably linked to a promoter sequence.

In some embodiments the DNA molecule includes two, three, or more gRNA sequences, each flanked by at least one Csy4 cleavage sequence.

In some embodiments, the promoter sequence is a RNA Polymerase II (Pol II) promoter or Pol III promoter, preferably a RNA Pol II promoter. In some embodiments the Pol II promoter is selected from the group consisting of CAG, EF1A, CAGGS, PGK, UbiC, CMV, B29, Desmin, Endoglin, FLT-1, GFPA, and SYN1 promoters.

In another aspect the invention provides a DNA molecule comprising a promoter sequence linked with one, two, three or more cassettes comprising: a sequence encoding a guide RNA, i.e., a sequence of about 100 nts, e.g., 95-300 nts, e.g., 95-105 nts for an *S*. *Pyogenes-based* system, linked to a Csy4 cleavage site, e.g., SEQ ID NO:1 or 2.

In some embodiments the DNA molecule comprises a Pol II promoter, operably linked to a first sequence encoding a first guide RNA linked to a Csy4 cleavage site, linked to a second sequence encoding a second guide RNA linked to a Csy4 cleavage site, linked to a third sequence encoding a third guide RNA linked to a Csy4 cleavage site. In some embodiments, further guide RNAs linked to Csy4 cleavage sites are included. For example, the DNA molecule can have the following structure:
Promoter - C4 - gRNA - C4 - gRNA - C4 - gRNA - C4
Promoter - C4 - gRNA - C4 - gRNA - C4 - gRNA - C4 - gRNA - C4
Promoter - C4 - gRNA - C4 - gRNA - C4 - gRNA - C4 - gRNA - C4 - gRNA C4
And so on. In this illustration C4 is a sequence encoding a Csy4 RNA cleavage site and gRNA is a sequence encoding a guide RNA.

In some embodiments, the the Cas9 sgRNA comprises the sequence:
(X₁₇₋₂₀) GUUUUAGAGCUAUGCUGUUUUG(X_{N}) (SEQ ID NO:4);
(X₁₇₋₂₀)GUUUUAGAGCUA (SEQ ID NO:5);
(X₁₇₋₂₀) GUUUUAGAGCUAUGCUGUUUUG (SEQ ID NO:6);
(X₁₇₋₂₀)GUUUUAGAGCUAUGCU (SEQ ID NO:7);
(X₁₇₋₂₀)GUUUUAGAGCUAGAAAUAGCAAGUUAAAAUAAGGCUAGUCCG (X_{N}) (SEQ ID NO:8);

(X₁₇₋₂₀)GUUUUAGAGCUAUGCUGAAAAGCAUAGCAAGUUAAAAUAAGGCUAGUC
CGUUAUC(X_{N}) (SEQ ID NO:9);

(X17-20)GUUUUAGAGCUAUGCUGUUUUGGAAACAAAACAGCAUAGCAAGUUAAA
AUAAGGCUAGUCCGUUAUC(XN) (SEQ ID NO:10);

(X17-20)GUUUUAGAGCUAGAAAUAGCAAGUUAAAAUAAGGCUAGUCCGUUAUCA
ACUUGAAAAAGUGGCACCGAGUCGGUGC(XN) (SEQ ID NO:11),

(X17-20)GUUUAAGAGCUAGAAAUAGCAAGUUUAAAUAAGGCUAGUCCGUUAUCA
ACUUGAAAAAGUGGCACCGAGUCGGUGC(SEQ ID NO:12);

(X₁₇₋₂₀)GUUUUAGAGCUAUGCUGGAAACAGCAUAGCAAGUUUAAAUAAGGCUAG UCCGUUAUCAACUUGAAAAAGUGGCACCGAGUCGGUGC (SEQ ID NO:13); or 
(X17-20)GUUUAAGAGCUAUGCUGGAAACAGCAUAGCAAGUUUAAAUAAGGCUAG
UCCGUUAUCAACUUGAAAAAGUGGCACCGAGUCGGUGC (SEQ ID NO:14),
wherein X₁₇₋₂₀ is a sequence complementary to the complementary strand of 17-20 consecutive nucleotides of a target sequence, preferably a target sequence immediately 5' of a protospacer adjacent motif (PAM), and X_{N} is any sequence that does not interfere with the binding of the ribonucleic acid to Cas9. Although a sequence of X₁₇₋₂₀ is exemplified herein with the *S. pyogenes* Cas9 system, longer sequences can also be used, e.g., as appropriate for other systems.

In some embodiments, the DNA molecule also includes a sequence encoding a functional Csy4 enzyme.

Also provided herein are vectors comprising the DNA molecules described herein, e.g., optionally comprising a sequence encoding a functional Csy4 enzyme. Also provided herein are the multiplex transcripts produced by the DNA molecules, e.g., intact RNAs that have not yet been cleaved with Csy4.

In yet another aspect, provided herein are methods for producing a plurality of guide RNAs in a cell. The methods include expressing the DNA molecules described herein in the cell.

In some embodiments, the cell is a mammalian cell, and the cell also expresses an exogenous functional Csy4 enzyme sequence, or the method further comprises administering a functional Csy4 enzyme or nucleic acid encoding a functional Csy4 enzyme.

In another aspect the invention provides methods for altering expression of one or a plurality of target genes in a cell. The methods include expressing a DNA molecule as described herein, e.g., a DNA molecule comprising a plurality of sequences encoding guide RNAs (gRNAs), wherein each gRNA comprises a variable sequence that is complementary to at least 17-20 nts of the one or more target genes, and each gRNA is flanked by at least one Csy4 cleavage sequence comprising or consisting of the sequence
GTTCACTGCCGTATAGGCAG (SEQ ID NO:1) or
GTTCACTGCCGTATAGGCAGCTAAGAAA (SEQ ID NO:2).

In the present methods and compositions, the gRNA can be either a single guide RNA comprising a fused tracrRNA and crRNA, as described herein, or can include just a crRNA, and the tracrRNA can be expressed from the same or a different DNA molecule. Thus in some embodiments the DNA molecules described herein also include a sequence encoding a tracrRNA. In some embodiments, the methods include expressing in the cells a separate tracrRNA, e.g., contacting the cells with a vector or DNA molecule that expresses a tracrRNA.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

### DESCRIPTION OF DRAWINGS

Figure 1 is a schematic illustrating constructs used in initial multiplex experiments, as follows:
   1 + 2: direct repeat crRNA array and Cas9, with separate tracrRNA
   3 + 4: short crRNA array separated by Csy4 sites with Csy4, Cas9, and separate tracrRNA
   5 + 6: full-length chimeric gRNAs separated by Csy4 site.
   7: nls-FLAG tagged Cas9
Figure 2 is a bar graph showing the results of experiments in cells expressing the constructs shown in Figure 1. The Csy4 site + full gRNA (constructs 5 and 6) was the most efficient multiplex framework.
Figure 3 is a schematic overview and comparison of exemplary standard and multiplex Csy4-based gRNA frameworks, and the transcripts they produce. Note that Csy4 enables the use of RNA Pol II promoters (e.g., CAG) as an alternative to U6, an RNA Pol III promoter.
Figure 4 is a bar graph showing that Csy4 cleaves a truncated recognition site producing gRNAs with higher activity in human cells. Processing of the truncated site also leaves a clean 5' end, effectively removing the 5' G restriction on gRNA target sequences imposed by the U6 promoter.
Figures 5A-C are sequences showing evidence of 2-target multiplex editing in single human cells. Individual deletions are observed at intended site 2 or 3. Multiple deletions on the same sequence are observed for sites 2 and 3. Deletions spanning sites 2 and 3 are also observed.
Figure 6 is a schematic showing successful multiplex expression of three gRNAs using the Csy4-based system.
Figure 7 is a bar graph showing gRNAs excised by Csy4 from RNAPol II-transcribed mRNA can efficiently recruit Cas9 nuclease to specific targets in human cells. In these experiments, gRNAs were expressed in longer mRNA transcripts made from the RNA Pol II CAG promoter.

### DETAILED DESCRIPTION

One potential advantage of the Cas9 system is the capability to recruit either nuclease activity or heterologous effector domains to more than one genomic locus or target site in a cell. However, such multiplex applications require the ability to efficiently express more than one gRNA in a cell. For mammalian cells, RNA polymerase III promoters (e.g., U6 promoter) have been used to express single short gRNAs. Previous attempts to express multiple gRNA components in human cells from a single transcript have not proven to be efficient.

Additional desirable capabilities for the Cas9 system would be to create inducible versions of the components and to enable tissue-specific expression of the components. RNA polymerase II promoters that are inducible and/or tissue-specific have been previously described. However, although Cas9 or dCas9 proteins could be expressed from such RNA pol II promoters, short, defined gRNAs cannot be expressed in this way as the start and stop sites of transcription from RNA pol II are imprecise. Indeed, to date, all gRNAs have been expressed from RNA polymerase III promoters, which are ideally suited for expression of short RNAs.

As demonstrated herein, Csy4, an endoribonuclease that recognizes a short RNA hairpin sequence, can be used to cleave out multiple functional gRNAs encoded on a single longer RNA transcript (produced from an RNA pol III promoter) cassette in which the individual gRNAs are separated by Csy4 cleavage sites. Functional gRNAs can be successfully cleaved from longer RNA transcripts expressed from an RNA pol II promoter.

### gRNA/Csy4 Multimeric Cassettes

Thus described herein are DNA molecules that encode longer RNA transcripts, referred to herein as multimeric cassettes, which include two or more gRNA sequences, wherein each gRNA is flanked by a Csy4 cleavage sequence. The DNA molecules can also include a promoter, and can optionally include one or more other transcription regulatory sequences, e.g., enhancers, silencers, insulators, and polyA sequences. See, e.g., Xu et al., Gene. 2001 Jul 11;272(1-2):149-56.

### Promoters

A number of promoters are known in the art that can be used in the present methods. In some embodiments, the promoter is a PolII or Pol III promoter, preferably a Pol II promoter. Various Pol II promoters have been described and can be used in the present compositions and methods, including the CAG promoter (see, e.g., Alexopoulou et al., BMC Cell Biology 9: 2, 2008; Miyazaki et al., Gene 79 (2): 269-77 (1989); Niwa et al., Gene 108 (2): 193-9 (1991); additional promoters include the EF1A, CAGGS, PGK, UbiC and CMV promoters, as well as tissue-specific promoters such as B29, Desmin, Endoglin, FLT-1, GFPA, SYN1, among others; sequences of numerous promoters are known in the art. For example, the CMV and PGK promoters can be amplified from pSicoR and pSicoR PGK respectively (Ventura et al., Proc Natl Acad Sci U S A 101: 10380-10385 (2004)), the UbiC promoter can be amplified from pDSLhpUGIH (ATCC), the CAGGS promoter can be amplified from pCAGGS (BCCM), and the EF1A promoter can be amplified from the pEF6 vector (Invitrogen). The Pol II core promoter is described in Butler and Kadonaga, Genes & Dev. 16: 2583-2592 (2002). Cleavage of the gRNAs out of a larger transcript driven by Pol II expression enables one to produce gRNAs that have any nucleotide at the 5'-most position (standard expression from a U6 or other RNA polymerase III promoter places restrictions on the identity of this nucleotide).

In some embodiments, a tissue-specific promoter is used, and a short, defined gRNA sequence can be processed out of the RNA-Pol II transcript.

A number of Pol III promoters are known in the art, including the U6 small nuclear (sn) RNA promoter, 7SK promoter, and the H1 promoter. See, e.g., Ro et al., BioTechniques, 38(4):625-627 (2005).

### Guide RNAs

Cas9 nuclease can be guided to specific genomic targets of at least 17-20 nts bearing an additional proximal protospacer adjacent motif (PAM) of sequence NGG by using a single gRNA bearing at least 17-20 nts at its 5' end that are complementary to the genomic DNA target site.

Thus, the compositions described herein can include a sequence encoding a single guide RNA (sgRNRA) comprising a crRNA fused to a normally trans-encoded tracrRNA, e.g., a single Cas9 guide RNA as described in Mali et al., Science 2013 Feb 15; 339(6121):823-6, with a sequence at the 5' end that is complementary to 17-20 nucleotides (nts) of a target sequence immediately 5' of a protospacer adjacent motif (PAM), e.g., NGG, NAG, or NNGG.

Methods of designing and expressing guide RNAs are known in the art. Guide RNAs generally speaking come in two different systems: 1) System 1 uses separate crRNA and tracrRNAs that function together to guide cleavage by Cas9; and 2) System 2 uses a chimeric crRNA-tracrRNA hybrid that combines the two separate guide RNAs in a single system (Jinek et al. 2012). The tracr-RNA can be variably truncated and a range of lengths has been shown to function in both the separate system (system 1) and the chimeric gRNA system (system 2). See, e.g., Jinek et al., Science 2012; 337:816-821; Mali et al., Science. 2013 Feb 15;339(6121):823-6; Cong et al., Science. 2013 Feb 15;339(6121):819-23; and Hwang and Fu et al., Nat Biotechnol. 2013 Mar;31(3):227-9; Jinek et al., Elife 2, e00471 (2013)). For System 2, generally the longer length chimeric gRNAs have shown greater on-target activity but the relative specificities of the various length gRNAs currently remain undefined and therefore it may be desirable in certain instances to use shorter gRNAs. In some embodiments, the gRNAs are complementary to a region that is within about 100-800 bp upstream of the transcription start site, e.g., is within about 500bp upstream of the transcription start site, includes the transcription start site, or within about 100-800 bp, e.g., within about 500 bp, downstream of the transcription start site. In some embodiments, vectors (e.g., plasmids) encoding more than one gRNA are used, e.g., plasmids encoding, 2, 3, 4, 5, or more gRNAs directed to different sites in the same region of the target gene. Additional guide RNAs, and methods of increasing the specificity of genome editing, are described in Provisional Patent Application Serial No. 61/838,178, entitled INCREASING

### SPECIFICITY FOR RNA-GUIDED GENOME EDITING

In some embodiments, the gRNA comprises or consists of the sequence:
(X₁₇₋₂₀) GUUUUAGAGCUAUGCUGUUUUG(X_{N}) (SEQ ID NO:4);
(X₁₇₋₂₀)GUUUUAGAGCUA (SEQ ID NO:5);
(X₁₇₋₂₀) GUUUUAGAGCUAUGCUGUUUUG (SEQ ID NO:6);
(X₁₇₋₂₀)GUUUUAGAGCUAUGCU (SEQ ID NO:7);
(X₁₇₋₂₀)GUUUUAGAGCUAGAAAUAGCAAGUUAAAAUAAGGCUAGUCCG (X_{N}) (SEQ ID NO:8);

(X17-20)GUUUUAGAGCUAUGCUGAAAAGCAUAGCAAGUUAAAAUAAGGCUAGUC

CGUUAUC(X_{N}) (SEQ ID NO:9);

(X17-20)GUUUUAGAGCUAUGCUGUUUUGGAAACAAAACAGCAUAGCAAGUUAAA
AUAAGGCUAGUCCGUUAUC(XN) (SEQ ID NO:10);

(X17-20)GUUUUAGAGCUAGAAAUAGCAAGUUAAAAUAAGGCUAGUCCGUUAUCA
ACUUGAAAAAGUGGCACCGAGUCGGUGC(XN) (SEQ ID NO:11),

(X17-20)GUUUAAGAGCUAGAAAUAGCAAGUUUAAAUAAGGCUAGUCCGUUAUCA
ACUUGAAAAAGUGGCACCGAGUCGGUGC(SEQ ID NO:12);

(X₁₇₋₂₀)GUUUUAGAGCUAUGCUGGAAACAGCAUAGCAAGUUUAAAUAAGGCUAG UCCGUUAUCAACUUGAAAAAGUGGCACCGAGUCGGUGC (SEQ ID NO:13); or 
(X17-20)GUUUAAGAGCUAUGCUGGAAACAGCAUAGCAAGUUUAAAUAAGGCUAG
UCCGUUAUCAACUUGAAAAAGUGGCACCGAGUCGGUGC (SEQ ID NO:14),
wherein X₁₇₋₂₀ is a sequence complementary to the complementary strand of at least 17-20 consecutive nucleotides of a target sequence (though in some embodiments this complementarity region may be longer than 20 nts, e.g., 21, 22, 23, 24, 25, 26, 27, 28, 29, or 30 or more nts, e.g., 17-30 nts), preferably a target sequence immediately 5' of a protospacer adjacent motif (PAM), e.g., NGG, NAG, or NNGG. X_{N} is any sequence, wherein N (in the RNA) can be 0-300 or 0-200, e.g., 0-100, 0-50, or 0-20, that does not interfere with the binding of the ribonucleic acid to Cas9. In some embodiments the RNA includes one or more U, e.g., 1 to 8 or more Us (e.g., U, UU, UUU, UUUU, UUUUU, UUUUUU, UUUUUUU, UUUUUUUU) at the 3' end of the molecule, as a result of the optional presence of one or more Ts used as a termination signal to terminate RNA PolIII transcription. In some embodiments the RNA includes one or more, e.g., up to 3, e.g., one, two, or three, or more additional nucleotides at the 5' end of the RNA molecule that is not complementary to the target sequence. Optionally, one or more of the RNA nucleotides is modified, e.g., locked (2'-O-4'-C methylene bridge), is 5'-methylcytidine, is 2'-O-methyl-pseudouridine, or in which the ribose phosphate backbone has been replaced by a polyamide chain, e.g., one or more of the nucleotides within the sequence X₁₇₋₂₀, one or more of the nucleotides within the sequence X_{N}, or one or more of the nucleotides within any sequence of the gRNA

For example, in some embodiments the chimeric guide RNAs described in Jinek et al. (Science. 337(6096):816-21 (2012)) can be used, e.g.,
(X₁₇₋₂₀₎GUUUUAGAGCUAGAAAUAGCAAGUUAAAAUAAGGCUAGUCCG (SEQ ID NO:8); (X₁₇₋₂₀₎GUUUUAGAGCUAGAAAUAGCAAGUUAAAAUAAGG (SEQ ID NO:9); in some embodiments, the sgRNA bearing a 5'-terminal 17-20-nucleotide sequence complementary to the target DNA sequence, and a 42-nucleotide 3'-terminal stem loop structure required for Cas9 binding described in Jinek et al., Elife. 2:e00471 (2013), e.g., (X₁₇₋₂₀₎GUUUUAGAGCUAGAAAUAGCAAGUUAAAAUAAGGCUAGUCCG (SEQ ID NO:8) are used.

In some embodiments, the guide RNA includes one or more Adenine (A) or Uracil (U) nucleotides on the 3' end.

Although the examples described herein utilize a single gRNA, the methods can also be used with dual gRNAs (e.g., the crRNA and tracrRNA found in naturally occurring systems). In this case, a single tracrRNA would be used in conjunction with multiple different crRNAs expressed using the present system, e.g., the following (note that for RNAs, T's are understood herein to be U's):
crRNA sequence: X₁₇₋₂₀-GTTTTAGAGCTAGAAA (SEQ ID NO:15)
tracrRNA sequence:
   TAGCAAGTTAAAATAAGGCTAGTCCGTTATCAACTTGAAAAAGTGGCACCGAGT CGGTGC (SEQ ID NO:16). In this case, the crRNA is used as the guide RNA in the methods and molecules described herein, and the tracrRNA can be expressed from the same or a different DNA molecule.

Furthermore, although guide RNAs having a sequence of 17-20 nucleotides of complementarity are exemplified herein, in some embodiments longer sequences can be used, e.g., 21, 22, 23, 24, 25, 26, 27, 28, 29, or 30 or more nts, e.g., 17-30 nts in place of 17-20 nts.

### Csy4 Cleavage Sequences

In the methods and compositions described herein, a Csy4 cleavage sequence is inserted into the DNA molecule such that each guide RNA is flanked by a cleavage sequence, with one or at least one cleavage sequence between each guide RNA. Exemplary Cys4 cleavage sequences include GTTCACTGCCGTATAGGCAG (truncated 20 nt) (SEQ ID NO:1) and GTTCACTGCCGTATAGGCAGCTAAGAAA (full 28 nt) (SEQ ID NO:2). As demonstrated herein, use of the truncated Csy4 cleavage site (SEQ ID NO:1) is more efficient in human cells than use of the standard site. To the best of the present inventors' knowledge, this is the first demonstration of Csy4 activity being utilized in human cells.

### Functional Csy4 Enzyme sequences

In the methods described herein, a functional Csy4 enzyme that is capable of cleaving the transcripts at the Csy4 cleavage sites, is also expressed in the cell.

Exemplary Csy4 sequences from Csy4 homologues from Pseudomonas aeruginosa UCBPP-PA14 (Pa14), Yersinia pestis AAM85295 (Yp), Escherichia coli UTI89 (Ec89), Dichelobacter nodosus VCS 1703A (Dn), Acinetobacter baumannii AB0057 (Ab), Moritella sp. PE36 (MP1, MP01), Shewanella sp. W3-18-1 (SW), Pasteurella multocida subsp. multocida Pm70 (Pm), Pectobacterium wasabiae (Pw), and Dickeya dadantii Ech703 (Dd) are set forth in Fig. S6 of Haurwitz et al., Science 329(5997): 1355-1358 (2010). In preferred embodiments, the Csy4 is from *Pseudomonas aeruginosa.*

In some embodiments, the Csy4 is also used to covalently link heterologous effector domains to the gRNAs. Csy4 is believed to be a single-turnover enzyme and remains bound to its target hairpin sequence after cleavage (Sternberg et al., RNA. 2012 Apr;18(4):661-72). Csy4 is thus expected to remain bound to the 3' end of each cleaved gRNA. Since as demonstrated herein the cleaved gRNAs appear to be functional in human cells, the presence of this Csy4 protein on the 3' end of the gRNA does not appear to affect the ability of the gRNA to complex with and direct Cas9 activity. Thus it is presumed that these gRNA-Csy4 fusions would also be able to direct Cas9 mutants that bear mutations that inactivate its catalytic nuclease activity (dCas9 proteins). Therefore, one could fuse heterologous functional domains (HFD) to Csy4 (Csy4-HFD), and a dCas9:sgRNA:Csy4-HFD complex could then direct such domains to a specific genomic locus. Examples of such HFD could include other nuclease domains such as that from Fokl, transcriptional activator or repressor domains, or other domains that modify histones or DNA methylation status.

The Csy4-HFD are created by fusing a heterologous functional domain (e.g., a transcriptional activation domain, e.g., from VP64 or NF-κB p65), to the N-terminus or C-terminus of a Csy4 protein, with or without an intervening linker, e.g., a linker of about 5-20 or 13-18 nucleotides. The transcriptional activation domains can be fused on the N or C terminus of the Csy4. In addition to transcriptional activation domains, other heterologous functional domains (e.g., transcriptional repressors (e.g., KRAB, SID, and others) or silencers such as Heterochromatin Protein 1 (HP1, also known as swi6), e.g., HP1α or HP1β; proteins or peptides that could recruit long non-coding RNAs (IncRNAs) fused to a fixed RNA binding sequence such as those bound by the MS2 coat protein, endoribonuclease Csy4, or the lambda N protein; enzymes that modify the methylation state of DNA (e.g., DNA methyltransferase (DNMT) or TET proteins); or enzymes that modify histone subunits (e.g., histone acetyltransferases (HAT), histone deacetylases (HDAC), histone methyltransferases (e.g., for methylation of lysine or arginine residues) or histone demethylases(e.g., for demethylation of lysine or arginine residues)) as are known in the art can also be used. A number of sequences for such domains are known in the art, e.g., a domain that catalyzes hydroxylation of methylated cytosines in DNA. Exemplary proteins include the Ten-Eleven-Translocation (TET)1-3 family, enzymes that converts 5-methylcytosine (5-mC) to 5-hydroxymethylcytosine (5-hmC) in DNA. See, e.g., WO/2014/144761.

**Sequences for human TET1-3 are known in the art and are shown in the following table:**

| | **GenBank Accession Nos.** | |
|---|---|---|
| Gene | Amino Acid | Nucleic Acid |
| TET1 | NP_085128.2 | NM 030625.2 |
| TET2^{∗} | NP_001120680.1 (var 1) | NM_001127208.2 |
| | NP_060098.3 (var 2) | NM_017628.4 |
| TET3 | NP_659430.1 | NM_144993.1 |

| | | |
|---|---|---|
| ^{∗} Variant (1) represents the longer transcript and encodes the longer isoform (a). Variant (2) differs in the 5' UTR and in the 3' UTR and coding sequence compared to variant 1. The resulting isoform (b) is shorter and has a distinct C-terminus compared to isoform a. | | |

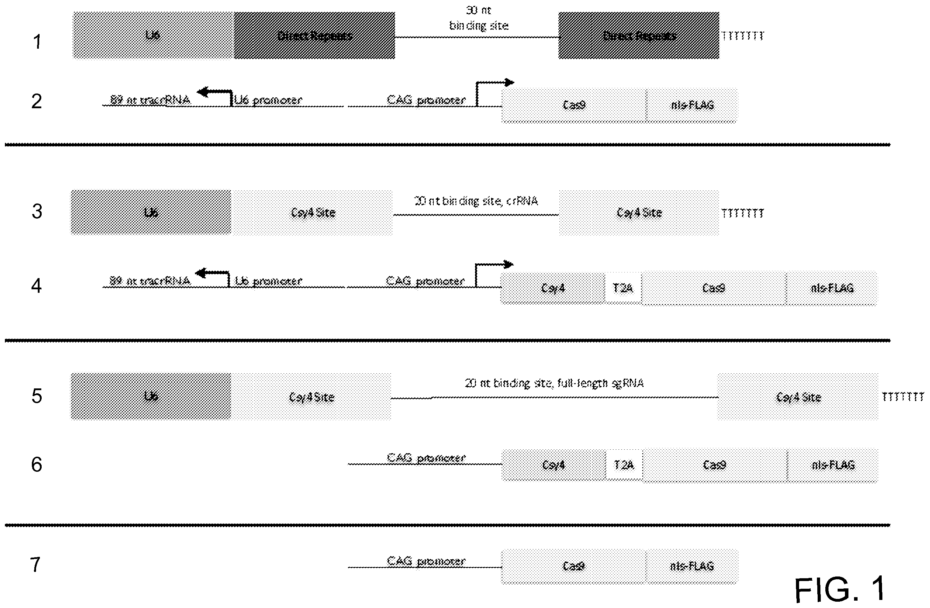

In some embodiments, all or part of the full-length sequence of the catalytic domain can be included, e.g., a catalytic module comprising the cysteine-rich extension and the 2OGFeDO domain encoded by 7 highly conserved exons, e.g., the Tet1 catalytic domain comprising amino acids 1580-2052, Tet2 comprising amino acids 1290-1905 and Tet3 comprising amino acids 966-1678. See, e.g., Fig. 1 of Iyer et al., Cell Cycle. 2009 Jun 1;8(11):1698-710. Epub 2009 Jun 27, for an alignment illustrating the key catalytic residues in all three Tet proteins, and the supplementary materials thereof (available at ftp site ftp.ncbi.nih.gov/pub/aravind/DONS/supplementary_material_DONS.html) for full length sequences (see, e.g., seq 2c); in some embodiments, the sequence includes amino acids 1418-2136 of Tet1 or the corresponding region in Tet2/3.

Other catalytic modules can be, e.g., from the proteins identified in Iyer et al., 2009.

In some embodiments, the fusion proteins include a linker between the Csy4 and the heterologous functional domains. Linkers that can be used in these fusion proteins (or between fusion proteins in a concatenated structure) can include any sequence that does not interfere with the function of the fusion proteins. In preferred embodiments, the linkers are short, e.g., 2-20 amino acids, and are typically flexible (i.e., comprising amino acids with a high degree of freedom such as glycine, alanine, and serine). In some embodiments, the linker comprises one or more units consisting of GGGS (SEQ ID NO:3) or GGGGS (SEQ ID NO:17), e.g., two, three, four, or more repeats of the GGGS (SEQ ID NO:3) or GGGGS (SEQ ID NO:17) unit. Other linker sequences can also be used, e.g., GGS, GGSG (SEQ ID NO:22), SGSETPGTSESA (SEQ ID NO:23), SGSETPGTSESATPES (SEQ ID NO:24), or SGSETPGTSESATPEGGSGGS (SEQ ID NO:25).

### Cas9

In the methods described herein, Cas9 is also expressed in the cells. A number of bacteria express Cas9 protein variants. The Cas9 from *Streptococcus pyogenes* is presently the most commonly used; some of the other Cas9 proteins have high levels of sequence identity with the *S. pyogenes* Cas9 and use the same guide RNAs. Others are more diverse, use different gRNAs, and recognize different PAM sequences as well (the 2-5 nucleotide sequence specified by the protein which is adjacent to the sequence specified by the RNA). Chylinski et al. classified Cas9 proteins from a large group of bacteria (RNA Biology 10:5, 1-12; 2013), and a large number of Cas9 proteins are listed in supplementary figure 1 and supplementary table 1 thereof, which are incorporated by reference herein. The constructs and methods described herein can include the use of any of those Cas9 proteins, and their corresponding guide RNAs or other guide RNAs that are compatible. The Cas9 from *Streptococcus thermophilus* LMD-9 CRISPR1 system has also been shown to function in human cells in Cong et al (Science 339, 819 (2013)). Additionally, Jinek et al. showed *in vitro* that Cas9 orthologs from S. *thermophilus* and *L. innocua,* (but not from *N. meningitidis* or *C. jejuni,* which likely use a different guide RNA), can be guided by a dual *S. pyogenes* gRNA to cleave target plasmid DNA, albeit with slightly decreased efficiency.

In some embodiments, the present system utilizes the Cas9 protein from *S. pyogenes,* either as encoded in bacteria or codon-optimized for expression in mammalian cells. An exemplary sequence of *Streptococcus pyogenes* Cas9 (residues 1-1368) fused to an HA epitope (amino acid sequence DAYPYDVPDYASL (SEQ ID NO: 18)) and a nuclear localization signal (amino acid sequence PKKKRKVEDPKKKRKVD (SEQ ID NO:19)) is as follows:

MDKKYSIGLDIGTNSVGWAVITDEYKVPSKKFKVLGNTDRHSIKKNLIGALLFDSGETAEATRLKRTARRRYTRRKNR
ICYLQEIFSNEMAKVDDSFFHRLEESFLVEEDKKHERHPIFGNIVDEVAYHEKYPTIYHLRKKLVDSTDKADLRLIYL
ALAHMIKFRGHFLIEGDLNPDNSDVDKLFIQLVQTYNQLFEENPINASGVDAKAILSARLSKSRRLENLIAQLPGEKKNG
LFGNLIALSLGLTPNFKSNFDLAEDAKLQLSKDTYDDDLDNLLAQIGDQYADLFLAAKNLSDAILLSDILRVNTEITKAP
LSASMIKRYDEHHQDLTLLKALVRQQLPEKYKEIFFDQSKNGYAGYIDGGASQEEFYKFIKPILEKMDGTEELLVKLNRE
DLLRKQRTFDNGSIPHQIHLGELHAILRRQEDFYPFLKDNREKIEKILTFRIPYYVGPLARGNSRFAWMTRKSEETITPW
NFEEVVDKGASAQSFIERMTNFDKNLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNFMQLIHDDSLTFKEDIQKAQVSGQ
GDSLHEHIANLAGSPAIKKGILQTVKVVDELVKVMGRHKPENIVIEMARENQTTQKGQKNSRERMKRIEEGIKELGSQIL
KEHPVENTQLQNEKLYLYYLQNGRDMYVDQELDINRLSDYDVDHIVPQSFLKDDSIDNKVLTRSDKNRGKSDNVPSEEVV

KKMKNYWRQLLNAKLITQRKFDNLTKAERGGLSELDKAGFIKRQLVETRQITKHVAQILDSRMNTKYDENDKLIREVKVI
TLKSKLVSDFRKDFQFYKVREINNYHHAHDAYLNAVVGTALIKKYPKLESEFVYGDYKVYDVRKMIAKSEQEIGKATAKY
FFYSNIMNFFKTEITLANGEIRKRPLIETNGETGEIVWDKGRDFATVRKVLSMPQVNIVKKTEVQTGGFSKESILPKRNS
DKLIARKKDWDPKKYGGFDSPTVAYSVLVVAKVEKGKSKKLKSVKELLGITIMERSSFEKNPIDFLEAKGYKEVKKDLII
KLPKYSLFELENGRKRMLASAGELQKGNELALPSKYVNFLYLASHYEKLKGSPEDNEQKQLFVEQHKHYLDEIIEQISEF
SKRVILADANLDKVLSAYNKHRDKPIREQAENIIHLFTLTNLGAPAAFKYFDTTIDRKRYTSTKEVLDATLIHQSITGLY
ETRIDLSQLGGDAYPYDVPDYASLGSGSPKKKRKVEDPKKKRKVD     (SEQ ID NO:20)

### See Jinek et al, 2013, supra.

In some embodiment, a Cas9 sequence is used that contains either of the D10A and H840A mutations to render the nuclease a nickase, or both the D10A and H840A mutations to render the nuclease portion of the protein catalytically inactive. The sequence of a catalytically inactive *S. pyogenes* Cas9 (dCas9) that can be used in the methods and compositions described herein is as follows; the mutations are in bold and underlined.

10 20 30 40 50 60
MDKKYSIGLA IGTNSVGWAV ITDEYKVPSK KFKVLGNTDR HSIKKNLIGA LLFDSGETAE

70 80 90 100 110 120
ATRLKRTARR RYTRRKNRIC YLQEIFSNEM AKVDDSFFHR LEESFLVEED KKHERHPIFG

130 140 150 160 170 180
NIVDEVAYHE KYPTIYHLRK KLVDSTDKAD LRLIYLALAH MIKFRGHFLI EGDLNPDNSD

190 200 210 220 230 240
VDKLFIQLVQ TYNQLFEENP INASGVDAKA ILSARLSKSR RLENLIAQLP GEKKNGLFGN

250 260 270 280 290 300
LIALSLGLTP NFKSNFDLAE DAKLQLSKDT YDDDLDNLLA QIGDQYADLF LAAKNLSDAI

310 320 330 340 350 360
LLSDILRVNT EITKAPLSAS MIKRYDEHHQ DLTLLKALVR QQLPEKYKEI FFDQSKNGYA

370 380 390 400 410 420
GYIDGGASQE EFYKFIKPIL EKMDGTEELL VKLNREDLLR KQRTFDNGSI PHQIHLGELH

430 440 450 460 470 480
AILRRQEDFY PFLKDNREKI EKILTFRIPY YVGPLARGNS RFAWMTRKSE ETITPWNFEE

490 500 510 520 530 540
VVDKGASAQS FIERMTNFDK NLPNEKVLPK HSLLYEYFTV YNELTKVKYV TEGMRKPAFL

550 560 570 580 590 600
SGEQKKAIVD LLFKTNRKVT VKQLKEDYFK KIECFDSVEI SGVEDRFNAS LGTYHDLLKI

610 620 630 640 650 660
IKDKDFLDNE ENEDILEDIV LTLTLFEDRE MIEERLKTYA HLFDDKVMKQ LKRRRYTGWG

670 680 690 700 710 720
RLSRKLINGI RDKQSGKTIL DFLKSDGFAN RNFMQLIHDD SLTFKEDIQK AQVSGQGDSL

730 740 750 760 770 780
HEHIANLAGS PAIKKGILQT VKVVDELVKV MGRHKPENIV IEMARENQTT QKGQKNSRER

790 800 810 820 830 840
MKRIEEGIKE LGSQILKEHP VENTQLQNEK LYLYYLQNGR DMYVDQELDI NRLSDYDVDA

850 860 870 880 890 900
IVPQSFLKDD SIDNKVLTRS DKNRGKSDNV PSEEVVKKMK NYWRQLLNAK LITQRKFDNL

910 920 930 940 950 960
TKAERGGLSE LDKAGFIKRQ LVETRQITKH VAQILDSRMN TKYDENDKLI REVKVITLKS

970 980 990 1000 1010 1020
KLVSDFRKDF QFYKVREINN YHHAHDAYLN AVVGTALIKK YPKLESEFVY GDYKVYDVRK

1030 1040 1050 1060 1070 1080
MIAKSEQEIG KATAKYFFYS NIMNFFKTEI TLANGEIRKR PLIETNGETG EIVWDKGRDF

1090 1100 1110 1120 1130 1140
ATVRKVLSMP QVNIVKKTEV QTGGFSKESI LPKRNSDKLI ARKKDWDPKK YGGFDSPTVA

1150 1160 1170 1180 1190 1200
YSVLVVAKVE KGKSKKLKSV KELLGITIME RSSFEKNPID FLEAKGYKEV KKDLIIKLPK

1210 1220 1230 1240 1250 1260
YSLFELENGR KRMLASAGEL QKGNELALPS KYVNFLYLAS HYEKLKGSPE DNEQKQLFVE

1270 1280 1290 1300 1310 1320
QHKHYLDEII EQISEFSKRV ILADANLDKV LSAYNKHRDK PIREQAENII HLFTLTNLGA

1330 1340 1350 1360
PAAFKYFDTT IDRKRYTSTK EVLDATLIHQ SITGLYETRI DLSQLGGD (SEQ ID NO:21)

See, e.g., Mali et al., 2013, *supra;* and Jinek et al., 2012, *supra.* Alternatively, the Cas9 can be a dCas9-heterofunctional domain fusion (dCas9-HFD) as described in U.S. Provisional Patent Application entitled RNA-GUIDED TARGETING OF GENETIC AND EPIGENOMIC REGULATORY PROTEINS TO SPECIFIC GENOMIC LOCI, filed on June 21, 2013 and assigned serial no. 61/838,148, and in PCT/US2014/027335.

The Cas9 can be expressed from an expression vector, as described herein, e.g., an extrachromosomal plasmid or viral vector comprising a sequence encoding Cas9, e.g., a Cas9 cDNA or gene; can be expressed from an exogenous Cas9 cDNA or gene that has integrated into the genome of the cell; an mRNA encoding Cas9; the actual Cas9 protein itself; or, in the case of non-mammalian cells, can be an exogenous Cas9.

### Expression Systems

Nucleic acid molecules comprising expression vectors can be used, e.g., for in vivo or in vitro expression of the Csy4/guide RNA constructs described herein. Vectors for expressing multiple gRNAs (potentially in an inducible or tissue-/cell-type specific fashion) can be used for research and therapeutic applications.

In order to use the fusion proteins and multimeric guide RNA cassettes described herein, it may be desirable to express them from a nucleic acid that encodes them. This can be performed in a variety of ways. For example, a nucleic acid encoding a guide RNA cassette or Csy4 or Cas9 protein can be cloned into an intermediate vector for transformation into prokaryotic or eukaryotic cells for replication and/or expression. Intermediate vectors are typically prokaryote vectors, e.g., plasmids, or shuttle vectors, or insect vectors, for storage or manipulation of the nucleic acid encoding the fusion protein or for production of the fusion protein. The nucleic acid encoding the guide RNA or fusion protein can also be cloned into an expression vector, for administration to a plant cell, animal cell, preferably a mammalian cell or a human cell, fungal cell, bacterial cell, or protozoan cell.

To obtain expression, a sequence encoding a guide RNA or fusion protein is typically subcloned into an expression vector that contains a promoter to direct transcription. Suitable bacterial and eukaryotic promoters are well known in the art and described, e.g., in Sambrook et al., Molecular Cloning, A Laboratory Manual (3d ed. 2001); Kriegler, Gene Transfer and Expression: A Laboratory Manual (1990); and Current Protocols in Molecular Biology (Ausubel et al., eds., 2010). Bacterial expression systems for expressing the engineered protein are available in, e.g., *E. coli, Bacillus* sp., and *Salmonella* (Palva et al., 1983, Gene 22:229-235). Kits for such expression systems are commercially available. Eukaryotic expression systems for mammalian cells, yeast, and insect cells are well known in the art and are also commercially available.

A number of suitable vectors are known in the art, e.g., viral vectors including recombinant retroviruses, lentivirus, adenovirus, adeno-associated virus, and herpes simplex virus 1, adenovirus-derived vectors, or recombinant bacterial or eukaryotic plasmids. For example, the expression construct can include: a coding region; a promoter sequence, e.g., a promoter sequence that restricts expression to a selected cell type, a conditional promoter, or a strong general promoter; an enhancer sequence; untranslated regulatory sequences, e.g., a 5'untranslated region (UTR), a 3'UTR; a polyadenylation site; and/or an insulator sequence. Such sequences are known in the art, and the skilled artisan would be able to select suitable sequences. See, e.g., Current Protocols in Molecular Biology, Ausubel, F.M. et al. (eds.) Greene Publishing Associates, (1989), Sections 9.10-9.14 and other standard laboratory manuals. In some embodiments, expression can be restricted to a particular cell type, using a tissue-specific promoter as is known in the art.

As described above, the vectors for expressing the guide RNAs can include RNA Pol II or Pol III promoters to drive expression of the guide RNAs. These human promoters allow for expression of gRNAs in mammalian cells following plasmid transfection. Alternatively, a T7 promoter may be used, e.g., for in vitro transcription, and the RNA can be transcribed in vitro and purified. The promoter used to direct expression of the nucleic acid depends on the particular application. For example, a strong constitutive promoter is typically used for expression and purification of fusion proteins. In contrast, when the fusion protein is to be administered in vivo for gene regulation, either a constitutive or an inducible promoter can be used, depending on the particular use of the fusion protein. In addition, a preferred promoter for administration of the fusion protein can be a weak promoter, such as HSV TK or a promoter having similar activity. The promoter can also include elements that are responsive to transactivation, e.g., hypoxia response elements, Gal4 response elements, lac repressor response element, and small molecule control systems such as tetracycline-regulated systems and the RU-486 system (see, e.g., Gossen & Bujard, 1992, Proc. Natl. Acad. Sci. USA, 89:5547; Oligino et al., 1998, Gene Ther., 5:491-496; Wang et al., 1997, Gene Ther., 4:432-441; Neering et al., 1996, Blood, 88:1147-55; and Rendahl et al., 1998, Nat. Biotechnol., 16:757-761).

In addition to the promoter, the expression vector typically contains a transcription unit or expression cassette that contains all the additional elements required for the expression of the nucleic acid in host cells, either prokaryotic or eukaryotic. A typical expression cassette thus contains a promoter operably linked, e.g., to the nucleic acid sequence encoding the fusion protein, and any signals required, e.g., for efficient polyadenylation of the transcript, transcriptional termination, ribosome binding sites, or translation termination. Additional elements of the cassette may include, e.g., enhancers, and heterologous spliced intronic signals.

The particular expression vector used to transport the genetic information into the cell is selected with regard to the intended use of the fusion protein, e.g., expression in plants, animals, bacteria, fungus, protozoa, etc. Standard bacterial expression vectors include plasmids such as pBR322 based plasmids, pSKF, pET23D, and commercially available tag-fusion expression systems such as GST and LacZ. A preferred tag-fusion protein is the maltose binding protein (MBP). Such tag-fusion proteins can be used for purification of the engineered TALE repeat protein. Epitope tags can also be added to recombinant proteins to provide convenient methods of isolation, for monitoring expression, and for monitoring cellular and subcellular localization, e.g., c-myc or FLAG.

Expression vectors containing regulatory elements from eukaryotic viruses are often used in eukaryotic expression vectors, e.g., SV40 vectors, papilloma virus vectors, and vectors derived from Epstein-Barr virus. Other exemplary eukaryotic vectors include pMSG, pAV009/A+, pMTO10/A+, pMAMneo-5, baculovirus pDSVE, and any other vector allowing expression of proteins under the direction of the SV40 early promoter, SV40 late promoter, metallothionein promoter, murine mammary tumor virus promoter, Rous sarcoma virus promoter, polyhedrin promoter, or other promoters shown effective for expression in eukaryotic cells.

Some expression systems have markers for selection of stably transfected cell lines such as thymidine kinase, hygromycin B phosphotransferase, and dihydrofolate reductase. High yield expression systems are also suitable, such as using a baculovirus vector in insect cells, with the fusion protein encoding sequence under the direction of the polyhedrin promoter or other strong baculovirus promoters.

The elements that are typically included in expression vectors also include a replicon that functions in *E. coli,* a gene encoding antibiotic resistance to permit selection of bacteria that harbor recombinant plasmids, and unique restriction sites in nonessential regions of the plasmid to allow insertion of recombinant sequences.

Standard transfection methods are used to produce bacterial, mammalian, yeast or insect cell lines that express large quantities of protein, which are then purified using standard techniques (see, e.g., Colley et al., 1989, J. Biol. Chem., 264:17619-22; Guide to Protein Purification, in Methods in Enzymology, vol. 182 (Deutscher, ed., 1990)). Transformation of eukaryotic and prokaryotic cells are performed according to standard techniques (see, e.g., Morrison, 1977, J. Bacteriol. 132:349-351; Clark-Curtiss & Curtiss, Methods in Enzymology 101:347-362 (Wu et al., eds, 1983).

Any of the known procedures for introducing foreign nucleotide sequences into host cells may be used. These include the use of calcium phosphate transfection, polybrene, protoplast fusion, electroporation, nucleofection, liposomes, microinjection, naked DNA, plasmid vectors, viral vectors, both episomal and integrative, and any of the other well-known methods for introducing cloned genomic DNA, cDNA, synthetic DNA or other foreign genetic material into a host cell (see, e.g., Sambrook et al., supra). It is only necessary that the particular genetic engineering procedure used be capable of successfully introducing at least one gene into the host cell capable of expressing the protein of choice.

In some embodiments, the Cas9 or Csy4 protein includes a nuclear localization domain which provides for the protein to be translocated to the nucleus. Several nuclear localization sequences (NLS) are known, and any suitable NLS can be used. For example, many NLSs have a plurality of basic amino acids, referred to as a bipartite basic repeats (reviewed in Garcia-Bustos et al, 1991, Biochim. Biophys. Acta, 1071:83-101). An NLS containing bipartite basic repeats can be placed in any portion of chimeric protein and results in the chimeric protein being localized inside the nucleus. In preferred embodiments a nuclear localization domain is incorporated into the final fusion protein, as the ultimate functions of the fusion proteins described herein will typically require the proteins to be localized in the nucleus. However, it may not be necessary to add a separate nuclear localization domain in cases where the protein has intrinsic nuclear translocation function.

The present invention includes the vectors and cells comprising the vectors.

### Libraries

Also provided herein are combinatorial libraries of gRNAs, e.g., in inducible, tissue- or cell-type specific multiplex vectors for research applications, e.g., for screening for potential drug targets or to define interactions at a genetic level.

### Methods of Use

The methods described can include expressing in a cell, or contacting the cell with, the multimeric cassettes as described herein, plus a nuclease that can be guided by the shortened gRNAs, e.g., a Cas9 nuclease as described above, and a Csy4 nuclease, as described above.

The described system is a useful and versatile tool for modifying the expression of multiple endogenous genes simultaneously, or for targeting multiple parts of a single gene. Current methods for achieving this require the use of a separate gRNA-encoding transcript for each site to be targeted. Separate gRNAs are not optimal for multiplex genome editing of cell populations as it is impossible to guarantee that each cell will express each gRNA; with multiple transcripts, cells get a complex and non-uniform random mixture of gRNAs. The present system, however, allows expression of multiple gRNAs from a single transcript, which allows targeting of multiple sites in the genome by expression of multiple gRNAs. Furthermore, with a single-transcript system, each cell should express all gRNAs with similar stoichiometry. This system could therefore easily be used to simultaneously alter expression of a large number of genes or to recruit multiple Cas9s or HFDs to a single gene, promoter, or enhancer. This capability will have broad utility, e.g., for basic biological research, where it can be used to study gene function and to manipulate the expression of multiple genes in a single pathway, and in synthetic biology, where it will enable researchers to create circuits in cell that are responsive to multiple input signals. The relative ease with which this technology can be implemented and adapted to multiplexing will make it a broadly useful technology with many wide-ranging applications.

The methods described herein include contacting cells with a nucleic acid encoding the multimeric gRNA cassettes described herein directed to one or more genes, and nucleic acids encoding Csy4 and Cas9, to thereby modulate expression of the one or more genes.

### EXAMPLES

The invention is further described in the following examples, which do not limit the scope of the invention described in the claims.

### Example 1. Multiplex Editing with CRISPR/Cas9

Three strategies were tried with the objective of making multiplex edits with CRISPR/Cas9 from arrays of either crRNAs or sgRNAs expressed from a single transcript, as follows:
1. direct-repeat flanked crRNA array and Cas9, with separate tracrRNA
2. short crRNA array separated by Csy4 sites, expressed with Csy4, Cas9, and separate tracrRNA
3. full-length single guide RNAs (sgRNAs) separated by Csy4 sites

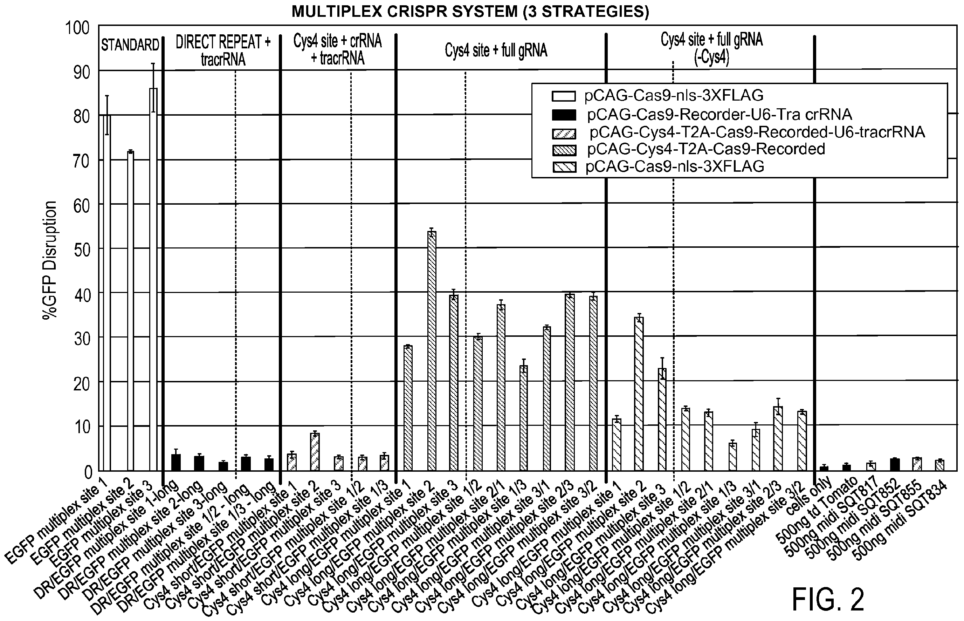

Each set of constructs (illustrated in Figure 1) was tested for the ability to efficiently disrupt EGFP in a U2OS-EGFP disruption assay. The results are shown in Figure 2. Constructs designed using strategies 1 and 2 exhibited the lowest activity in the EGFP-disruption assay even for single targets; therefore further experiments (described below) focused on optimizing strategy 3.

### Example 2. Multiplex Expression of Highly Active CRISPR Guide RNAs from RNA Polymerase II and III Promoters in Mammalian Cells

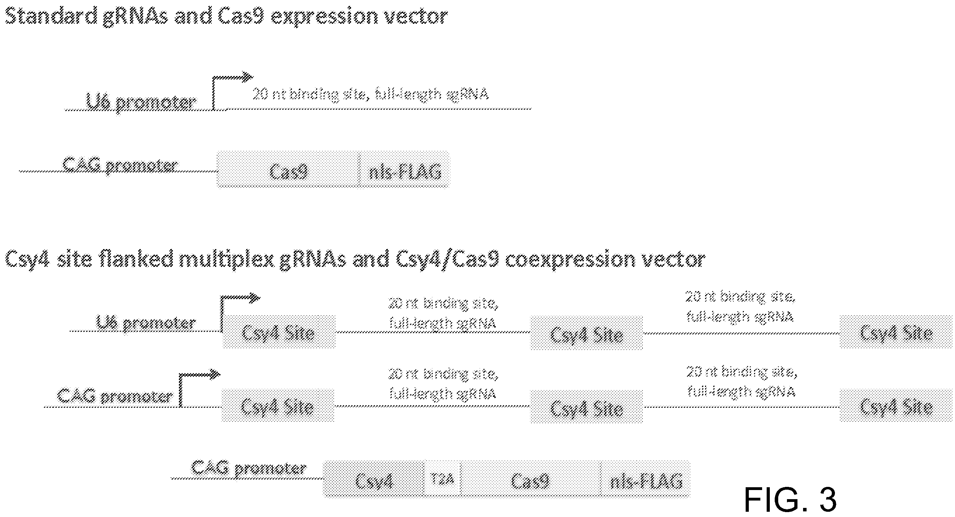

A schematic overview of an exemplary strategy for cleaving gRNAs out from longer transcripts using the Csy4 nuclease is shown in Figure 3. In initial experiments to demonstrate proof-of-concept, two versions of the Csy4-cleaved RNA hairpin site were tested for cleavage in human cells. To do this, gRNAs flanked by one of two Csy4 cleavage sites were expressed:
1. GTTCACTGCCGTATAGGCAGCTAAGAAA (full 28 nt) (SEQ ID NO:2)
2. GTTCACTGCCGTATAGGCAG (truncated 20 nt) (SEQ ID NO:1)

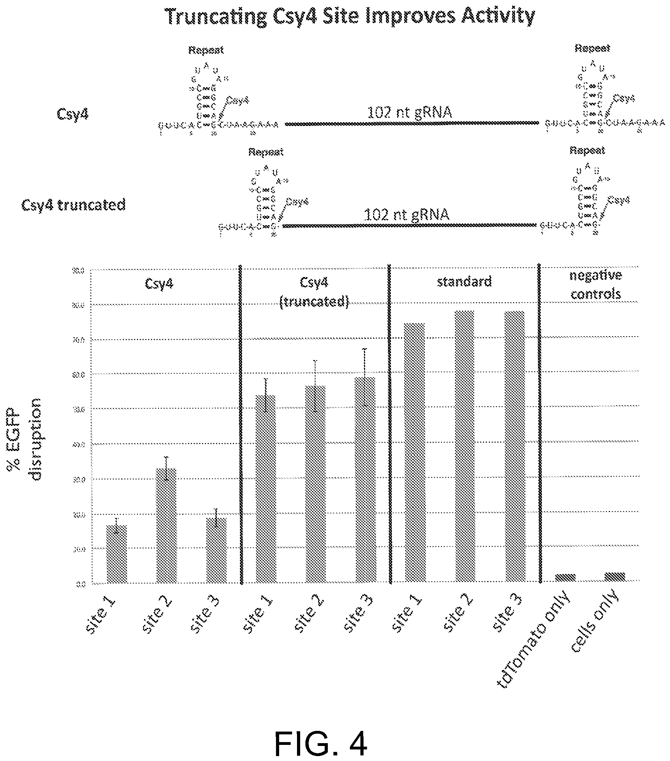

The results showed that gRNAs flanked on their 5' and 3' ends with the truncated 20 nt sequence were more active in mammalian cells than those flanked by the longer 28 nt sequence (Figure 4). To the best of the present inventors' knowledge, this is the first demonstration that Csy4 nuclease can be used to process RNA transcripts in live human cells. One important additional advantage of the 20 nt truncated site is that, unlike the longer 28 nt sequence, it does not leave any additional nucleotides on the 5' end of a gRNA processed from the longer transcript (Figure 4). This enables expression of gRNAs that have ANY desired nucleotide at the 5'-most position. This is an improvement relative to expression of gRNAs from RNA polymerase III promoters which have a requirement for specific nucleotide(s) at the 5'-most position.

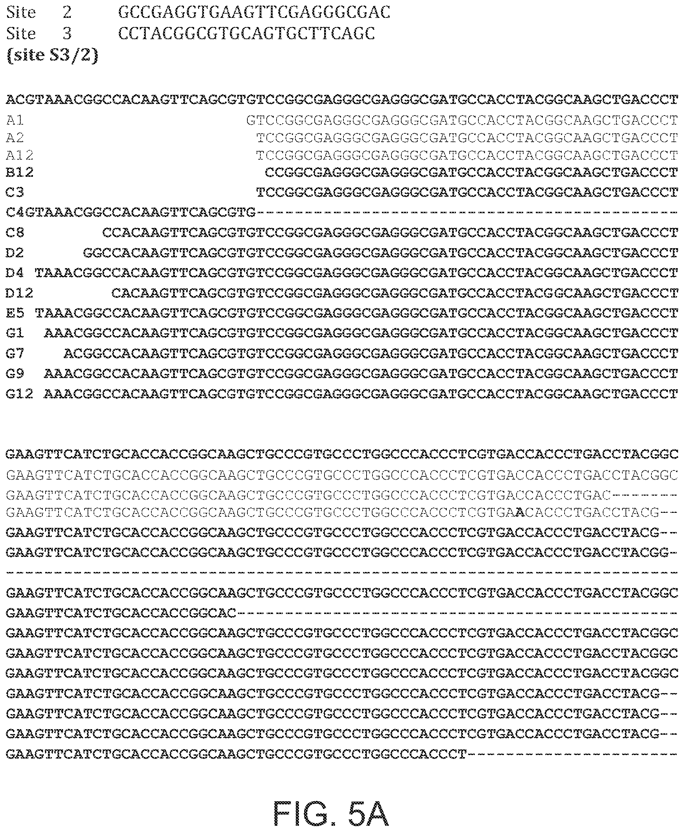

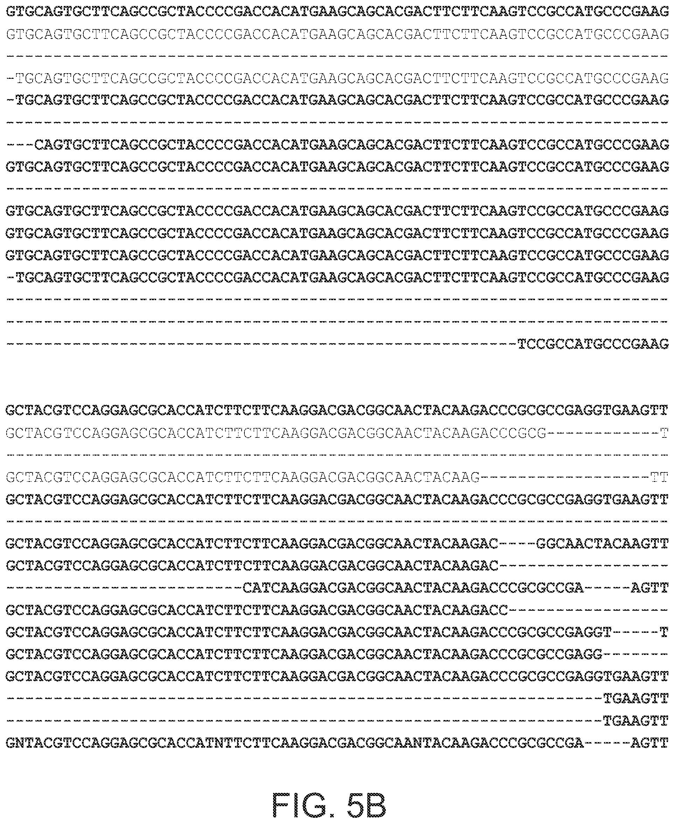

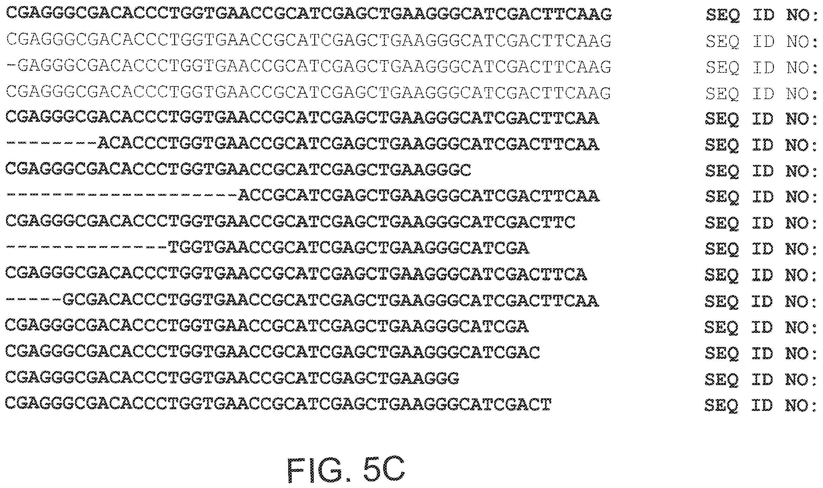

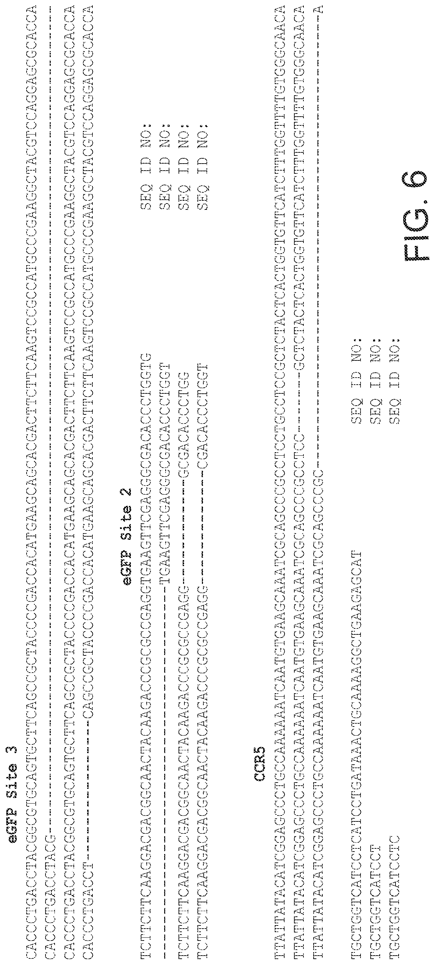

Using this Csy4-based system, the efficient expression of two and three different gRNAs (Figures 5 and 6) was demonstrated. gRNAs simultaneously expressed using this approach induced alterations at the expected sites in human cells.

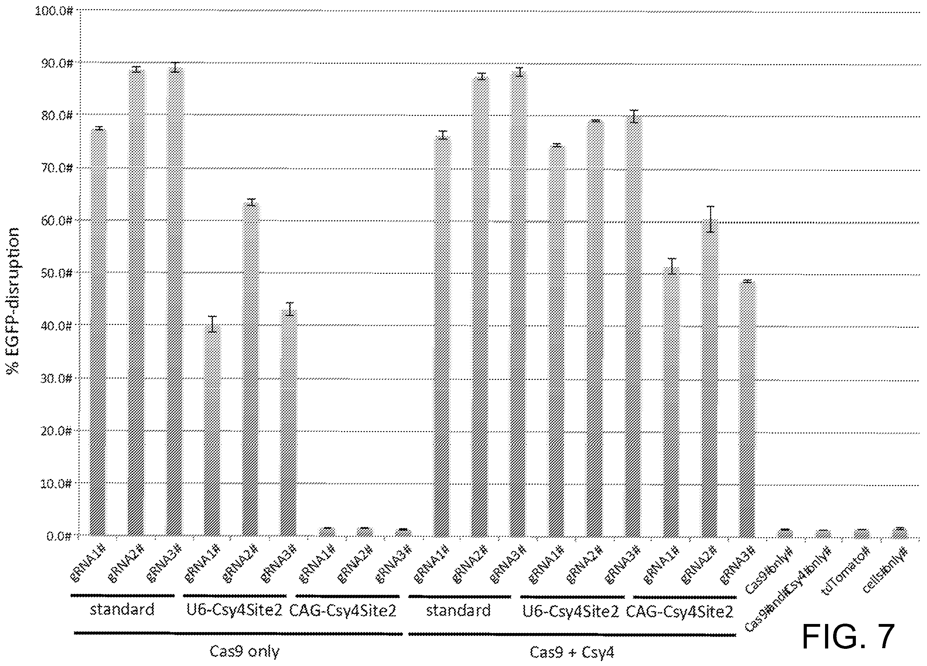

These results also demonstrated that this Csy4-based strategy could be used with gRNAs encoded on a longer mRNA produced by an RNA Pol II promoter (Figure 7). In these experiments, one of three different single gRNAs flanked by the truncated Csy4 sites was encoded on an mRNA produced by the CAG promoter (an RNA Pol II promoter). As shown in Figure 7, all three of these constructs could produce functional gRNA that could direct Cas9 nuclease in human cells but only in the presence of Csy4. The level of targeted Cas9 activity observed was comparable to (albeit somewhat lower than) what is observed when these gRNAs are expressed singly using a standard RNA Pol III promoter or as a Csy4-flanked transcript from an RNA Pol III promoter (Figure 7).

In summary, the present results demonstrated that: 1) up to three functional gRNAs can be produced from a single RNA pol III transcript when separated by Csy4 cleavage sites and in the presence of Csy4 in human cells, 2) multiple Csy4-processed gRNAs can be used to direct Cas9 nuclease to introduce multiplex changes in a single human cell, and 3) a functional gRNA flanked by Csy4 cleavage sites can be excised by Csy4 nuclease from a longer mRNA transcript made from an RNA polymerase II promoter.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### The invention will now be defined by reference to the following clauses:

1. A deoxyribonucleic acid (DNA) molecule comprising:
   a plurality of sequences encoding guide RNAs (gRNAs), wherein each gRNA is flanked by at least one Csy4 cleavage sequence comprising or consisting of the sequence GTTCACTGCCGTATAGGCAG (SEQ ID NO:1) or GTTCACTGCCGTATAGGCAGCTAAGAAA (SEQ ID NO:2).
2. The DNA molecule of clause 1, operably linked to a promoter sequence.
3. The DNA molecule of clause 1, comprising two, three, or more gRNA sequences.
4. The DNA molecule of clause 1, wherein the promoter sequence is a RNA Polymerase II (Pol II) promoter or Pol III promoter.
5. The DNA molecule of clause 1, wherein the promoter sequence is a RNA Pol II promoter.
6. The DNA molecule of clause 5, wherein the Pol II promoter is selected from the group consisting of CAG, EF1A, CAGGS, PGK, UbiC, CMV, B29, Desmin, Endoglin, FLT-1, GFPA, and SYN1 promoters.
7. A DNA molecule comprising a promoter sequence linked with one, two, three or more cassettes comprising: a sequence encoding a guide RNA (gRNA), linked to a Csy4 cleavage site.
8. The DNA molecule of clause 7, comprising:
   a Pol II promoter, operably linked to a first sequence encoding a first guide RNA linked to a Csy4 cleavage site, linked to a second sequence encoding a second guide RNA linked to a Csy4 cleavage site, linked to a third sequence encoding a third guide RNA linked to a Csy4 cleavage site.
9. The DNA molecule of clause 7, comprising a guide RNA sequence of about 100 nts.
10. The DNA molecule of clause 7, comprising a Csy4 cleavage site of SEQ ID NO: 1 or 2.
11. The DNA molecule of clause 1 or 7, further comprising a sequence encoding a functional Csy4 enzyme.
12. The DNA molecule of clause 1 or clause 7, wherein the gRNA comprises the sequence:
   (X₁₇₋₂₀) GUUUUAGAGCUAUGCUGUUUUG(X_{N}) (SEQ ID NO:4);
   (X₁₇₋₂₀)GUUUUAGAGCUA (SEQ ID NO:5);
   (X₁₇₋₂₀) GUUUUAGAGCUAUGCUGUUUUG (SEQ ID NO:6);
   (X₁₇₋₂₀)GUUUUAGAGCUAUGCU (SEQ ID NO:7);

(X17-20)GUUUUAGAGCUAGAAAUAGCAAGUUAAAAUAAGGCUAGUCCG
(XN) (SEQ ID NO:8);

(X17-20)GUUUUAGAGCUAUGCUGAAAAGCAUAGCAAGUUAAAAUAAGG
CUAGUCCGUUAUC(XN) (SEQ ID NO:9);

(X17-20)GUUUUAGAGCUAUGCUGUUUUGGAAACAAAACAGCAUAGCAA
GUUAAAAUAAGGCUAGUCCGUUAUC(XN) (SEQ ID NO:10);

(X17-20)GUUUUAGAGCUAGAAAUAGCAAGUUAAAAUAAGGCUAGUCCG
UUAUCAACUUGAAAAAGUGGCACCGAGUCGGUGC(XN) (SEQ ID
NO:11),

(X17-20)GUUUAAGAGCUAGAAAUAGCAAGUUUAAAUAAGGCUAGUCCG
UUAUCAACUUGAAAAAGUGGCACCGAGUCGGUGC(SEQ ID NO:12);

   (X₁₇₋₂₀)GUUUUAGAGCUAUGCUGGAAACAGCAUAGCAAGUUUAAAUAA GGCUAGUCCGUUAUCAACUUGAAAAAGUGGCACCGAGUCGGUGC (SEQ ID NO:13); or 
(X17-20)GUUUAAGAGCUAUGCUGGAAACAGCAUAGCAAGUUUAAAUAA
GGCUAGUCCGUUAUCAACUUGAAAAAGUGGCACCGAGUCGGUGC
(SEQ ID NO:14),
   wherein X₁₇₋₂₀ is a sequence complementary to the complementary strand of 17-20 consecutive nucleotides of a target sequence, preferably a target sequence immediately 5' of a protospacer adjacent motif (PAM), and X_{N} is any sequence that does not interfere with the binding of the ribonucleic acid to Cas9.
13. The DNA molecule of clause 1 or clause 7, comprising one or more U at the 3' end of the molecule.
14. An RNA molecule encoded by the DNA molecule of clauses 1-13
15. A vector comprising the DNA molecule of clauses 1-13.
16. The vector of clause 15, further comprising a sequence encoding a functional Csy4 enzyme.
17. A method of producing a plurality of guide RNAs in a cell, the method comprising expressing the DNA molecule of clauses 1-13 in the cell, or contacting the cell with the RNA molecule of clause 14.
18. The method of clause 17, wherein the cell is a mammalian cell and the cell also expresses an exogenous functional Csy4 enzyme sequence, or the method further comprises administering a functional Csy4 enzyme or nucleic acid encoding a functional Csy4 enzyme.
19. A method of altering expression of a plurality of target genes in a cell, the method comprising expressing a DNA molecule comprising a plurality of sequences encoding guide RNAs (gRNAs), wherein each gRNA comprises a sequence that is complementary to at least 17-20 nts of a target gene, and each gRNA is flanked by at least one Csy4 cleavage sequence comprising or consisting of the sequence GTTCACTGCCGTATAGGCAG (SEQ ID NO: 1) or GTTCACTGCCGTATAGGCAGCTAAGAAA (SEQ ID NO:2).
20. A method of altering expression of a target gene in a cell, the method comprising expressing a DNA molecule comprising a plurality of sequences encoding guide RNAs (gRNAs), wherein each gRNA comprises a sequence that is complementary to at least 17-20 nts of the target gene, and each gRNA is flanked by at least one Csy4 cleavage sequence comprising or consisting of the sequence GTTCACTGCCGTATAGGCAG (SEQ ID NO: 1) or GTTCACTGCCGTATAGGCAGCTAAGAAA (SEQ ID NO:2).

## Claims

1. A deoxyribonucleic acid (DNA) molecule comprising:
a plurality of sequences encoding crRNAs, wherein each crRNA is flanked by at least one Csy4 cleavage sequence. and a sequence encoding a tracrRNA.

2. The DNA molecule of claim 1, wherein the at least one Csy4 cleavage sequence consists of the sequence GTTCACTGCCGTATAGGCAG (SEQ ID NO: 1).

3. The DNA molecule of claim 1 or 2, operably linked to a promoter sequence, and/or, comprising two, three, or more crRNA sequences, and/or, The DNA molecule of claim 2, operably linked to a promoter sequence, wherein the promoter sequence is a RNA Polymerase II (Pol II) promoter or Pol III promoter, preferably, wherein the promoter sequence is a RNA Pol II promoter, preferably, wherein the Pol II promoter is selected from the group consisting of CAG, EF1A, CAGGS, PGK, UbiC, CMV, B29, Desmin, Endoglin, FLT-1, GFPA, and SYN1 promoters.

4. The DNA molecule of claim 1, wherein the plurality of sequences are one, two, three or more cassettes comprising: a sequence encoding a crRNA, linked to a Csy4 cleavage site and a sequence encoding a tracrRNA and further comprising a promoter sequence linked with the one, two, three or more cassettes.

5. The DNA molecule of claim 4, wherein the Csy4 cleavage site consists of the sequence GTTCACTGCCGTATAGGCAG (SEQ ID NO:1) and/or wherein the DNA molecule comprises: a Pol II promoter, operably linked to a first sequence encoding a first crRNA linked to a Csy4 cleavage site, linked to a second sequence encoding a second crRNA linked to a Csy4 cleavage site, linked to a third sequence encoding a third crRNA linked to a Csy4 cleavage site.

6. The DNA molecule of any one of claims 1-5, comprising a crRNA sequence of about 100 nts, and/or, further comprising a sequence encoding a functional Csy4 enzyme.

7. The DNA molecule of any one of claims 1-6, wherein
the crRNA sequences comprise: (X₁₇₋₂₀) GTTTTAGAGCTAGAAA (SEQ ID NO: 15); and the
tracrRNA sequence comprises: 
TAGCAAGTTAAAATAAGGCTAGTCCGTTATCAACTTGAAAAAGTGGCACCGAGT
CGGTGC (SEQ ID NO: 16);
 wherein X₁₇₋₂₀ is a sequence complementary to the complementary strand of 17-20 consecutive nucleotides of a target sequence, preferably a target sequence immediately 5' of a protospacer adjacent motif (PAM).

8. The DNA molecule of any one of claims 1-7, comprising one or more U at the 3' end of the molecule.

9. An RNA molecule encoded by the DNA molecule of any one of claims 1-8.

10. A vector comprising the DNA molecule of any one of claims 1-8.

11. The vector of claim 10, further comprising a sequence encoding a functional Csy4 enzyme.

12. A method of producing a plurality of crRNAs in a cell, the method comprising contacting the cell *in vitro* with the RNA molecule of claim 9.

13. The method of claim 12, wherein the cell is a mammalian cell and the cell also expresses an exogenous functional Csy4 enzyme sequence.

14. A method of altering expression of a target gene or a plurality of target genes in a cell, the method comprising contacting the cell *in vitro* with the DNA molecule of any one of claims 1-8 or the RNA molecule of claim 9, wherein each crRNA comprises a sequence that is complementary to at least 17-20 nts of a target gene.

15. Use of the DNA molecule of any one of claims 1-8 or the RNA molecule of claim 9 for altering expression of a target gene or a plurality of target genes in a cell, wherein each crRNA comprises a sequence that is complementary to at least 17-20 nts of a target gene.
